(19) **Europäisches Patentamt / European Patent Office / Office européen des brevets**

(11) **EP 4 609 903 A2**

(12) # EUROPEAN PATENT APPLICATION

(43) Date of publication:
**03.09.2025 Bulletin 2025/36**

(21) Application number: **25170495.3**

(22) Date of filing: **29.06.2021**

(51) International Patent Classification (IPC):
***A61M 16/16*** ^(2006.01)

(52) Cooperative Patent Classification (CPC):
**A61M 16/024; A61M 16/0683;** A61B 5/0826;
A61B 5/4806; A61B 5/4818; A61M 16/0616;
A61M 16/16; A61M 2016/0027; A61M 2205/0205;
A61M 2205/18; A61M 2205/332; A61M 2205/3375;
A61M 2205/42; A61M 2205/505; A61M 2205/581;
(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2020 SG 10202006315Y**

(62) Document number(s) of the earlier application(s) in
accordance with Art. 76 EPC:
**21834614.6 / 4 171 697**

(71) Applicant: **ResMed Asia Pte. Ltd.
Singapore 639443 (SG)**

(72) Inventor: **VALIYAMBATH, Mohankumar Krishan
Singapore 509016 (SG)**

(74) Representative: **Vossius & Partner
Patentanwälte Rechtsanwälte mbB
Siebertstrasse 3
81675 München (DE)**

Remarks:
This application was filed on 14-04-2025 as a
divisional application to the application mentioned
under INID code 62.

(54) **A PATIENT INTERFACE AND A POSITIONING AND STABILISING STRUCTURE FOR A PATIENT INTERFACE**

(57)    The present invention relates to a positioning and stabilising structure for a patient interface, comprising: a headband formed at least partly from a textile material and having an upper textile portion movably connected to a first lower textile portion, the headband comprising at least one sensor provided in or on the upper textile portion and/or the first lower textile portion; wherein the headband is wearable on a patient's head in a first configuration in which the first lower textile portion is proximate to the upper textile portion and configured to overlay the patient's forehead, and a second configuration in which the first lower textile portion is separated from the upper textile portion and provides a force to hold a seal-forming structure of the patient interface in a therapeutically effective position on the patient's head, the first lower textile portion configured to overlay the patient's cheeks in the second configuration.

EP 4 609 903 A2

(52) Cooperative Patent Classification (CPC): (Cont.)
A61M 2205/582; A61M 2230/10; A61M 2230/14;
A61M 2230/205; A61M 2230/432; A61M 2230/50;
A61M 2230/60; A61M 2230/63; G16H 20/40;
Y02A 90/10

**Description**

1 CROSS-REFERENCE TO RELATED APPLICATIONS

The present application claims the benefit of Singapore Patent Application No. 10202006315Y, filed on 30 June 2020, the entire contents of which are incorporated by reference.

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

2.2 DESCRIPTION OF THE RELATED ART

2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or

washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched air at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

**2.2.2.3 Supplementary oxygen**

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

**2.2.3 Respiratory Therapy Systems**

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

**2.2.3.1 Patient Interface**

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH$_2$O relative to ambient pressure. For other forms of therapy, such as the delivery of oxygen, the patient interface may not include a seal sufficient to facilitate delivery to the airways of a supply of gas at a positive pressure of about 10 cmH$_2$O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of being obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use, a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

*2.2.3.1.1 Seal-forming structure*

Patient interfaces may include a seal-forming structure. Since it is in direct contact with the patient's face, the shape and configuration of the seal-forming structure can have a direct impact on the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to, for example, overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design can fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Limited, describe examples of nasal pillows masks: International Patent Application WO2004/073,778 (describing amongst other things aspects of the ResMed Limited SWIFT™ nasal pillows), US Patent Application 2009/0044808 (describing amongst other things aspects of the ResMed Limited SWIFT™ LT nasal pillows); International Patent Applications WO 2005/063,328 and WO 2006/130,903 (describing amongst other things aspects of the ResMed Limited MIRAGE LIBERTY™ full-face mask); International Patent Application WO 2009/052,560 (describing amongst other things aspects of the ResMed Limited SWIFT™ FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

A further difficulty with known positioning and stabilising arrangements for respiratory therapy is that they can be complicated for patients to attach and to correctly position on the head for effective therapy. Additionally, for those who are new to respiratory therapy, the wearing of an unfamiliar device that may cover a substantial part of the head can be an unfamiliar and confronting experience, which may discourage patient compliance.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH$_2$O).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the onset of inhalation. This therapy mode is known as pulsed oxygen delivery (POD) or demand mode, in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system. For example, it would be useful to have performance data, such as data indicative of the effects of therapy on a patient, and/or data indicative of functioning of the patient interface, to enable greater control to be exercised over therapy.

Existing processes to communicate and manage such data can be one or more of costly, time-consuming, and error-prone.

### 2.2.3.7 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH$_2$O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage™ | nasal | 36 (3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage™ SoftGel | nasal | 29 (3) | 22 (3) | 2008 |
| ResMed Mirage™ FX | nasal | 26 (3) | 18 (3) | 2010 |
| ResMed Mirage Swift™ (*) | nasal pillows | 37 | 29 | 2004 |
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |
| (* one specimen only, measured using test method specified in ISO 3744 in CPAP mode at 10 cmH$_2$O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing due to the complex equipment required, which is difficult or even impossible for patients to attach correctly for a proper assessment to occur.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

## 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a patient interface comprising:

a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient,

a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and

a positioning and stabilising structure to provide a force to hold the seal-forming structure in a therapeutically effective position on the patient's head.

One form of the present technology comprises a positioning and stabilising structure for a patient interface that is convertible between a first, pre-therapy, configuration in which it can be worn by a patient as a headband, and a second configuration in which it can be used to apply a force to maintain a seal-forming structure in position on the patient's face for effective respiratory therapy.

Another form of the present technology comprises a patient interface having one or more sensors embedded, attached or otherwise disposed therein and/or thereon for measuring patient data and/or equipment-related data for screening, monitoring and/or diagnostic purposes.

One form of the present technology comprises a positioning and stabilising structure for a patient interface, comprising: an upper textile portion comprising a resilient circumferential band for fitting to a patient's head in use; and at least one lower textile portion movably (e.g., hingedly) connected to the upper textile portion; wherein at least a first lower textile portion is stretchable relative to the upper textile portion, and is constructed and arranged to provide a force to hold a seal-forming structure of the patient interface in a therapeutically effective position on the patient's head.

One form of the present technology comprises a patient interface comprising:

a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient,

a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use;

a positioning and stabilising structure to provide a force to hold the seal-forming structure in a therapeutically effective position on the patient's head;

wherein the positioning and stabilising structure comprises:

an upper textile portion comprising a resilient circumferential band for fitting to a patient's head in use; and
at least one lower textile portion movably connected to the upper textile portion;
wherein at least a first lower textile portion is stretchable relative to the upper textile portion, and is constructed and arranged to provide a force to hold a seal-forming structure of the patient interface in a therapeutically effective position on the patient's head.

One form of the present technology comprises a positioning and stabilising structure for a patient interface, comprising: a front section and a rear section forming a continuous loop of material, the front section forming a first bifurcated section with a first part and a second part; an upper textile portion comprising a resilient circumferential band for fitting to a patient's head in use, the upper textile portion formed from the rear section and from the first part; and at least one lower textile portion of the at least one lower textile portion movably connected to the upper textile portion; wherein at least a first lower textile portion is stretchable relative to the upper textile portion, and is constructed and arranged to provide a force to hold a seal-forming structure of the patient interface in a therapeutically effective position on the patient's head; and wherein the first lower textile portion is movable between a first position and a second position, the first lower textile portion configured to be proximate to the first part and to overlay the patient's frontal bone in the first position, and the first lower textile portion configured to be distal to the first part and overlay the patient's cheeks in the second position.

One form of the present technology comprises a patient interface comprising:

a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient,
a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use;
a positioning and stabilising structure to provide a force to hold the seal-forming structure in a therapeutically effective position on the patient's head;
wherein the positioning and stabilising structure comprises:

a front section and a rear section forming a continuous loop of material, the front section forming a first bifurcated section with a first part and a second part;
an upper textile portion comprising a resilient circumferential band for fitting to a patient's head in use, the upper textile portion formed from the rear section and from the first part; and
at least one lower textile portion movably connected to the upper textile portion;
wherein at least a first lower textile portion of the at least one lower textile portion is stretchable relative to the upper textile portion, and is constructed and arranged to provide a force to hold a seal-forming structure of the patient interface in a therapeutically effective position on the patient's head; and
wherein the first lower textile portion is movable between a first position and a second position, the first lower textile portion configured to be proximate to the first part and to overlay the patient's frontal bone in the first position, and the first lower textile portion configured to be distal to the first part and overlay the patient's cheeks in the second position.

In examples of the preceding aspects: (a) the first lower textile portion is integral with the upper textile portion; (b) the positioning and stabilising structure is in the form of a headband; (c) the headband comprises a first bifurcated section comprising a first part of the upper textile portion and further comprising the first lower textile portion, the first bifurcated section being located at a front of the headband; (d) the headband comprises a second bifurcated section comprising a second part of the upper textile portion and further comprising a second lower textile portion, the second bifurcated section being located at a rear of the headband; (e) the first lower textile portion is a seal retention band that is resiliently stretchable along at least part of its length, and is adapted to engage with an outer surface of the patient interface to hold the seal-forming structure in the therapeutically effective position; (f) the seal retention band is more stretchable than the upper textile portion; the seal retention band is adapted to be received in a channel of the patient interface; (g) the channel is formed in a plenum chamber of the patient interface; and/or (h) the seal retention band comprises a port for coupling the seal-forming structure to an air circuit for supplying pressurised air to the patient.

In examples of the preceding aspects: (a) the positioning and stabilising structure comprises a pair of lower textile portions adapted to couple to each other, and/or to an intermediate structure, to provide said force; (b) the intermediate structure is a harness that, in use, retains the seal-forming structure in the therapeutically effective position; (c) the intermediate structure comprises the seal-forming structure, or part thereof; and/or (d) the lower textile portions couple to each other, or to the intermediate structure, by one or more resilient hooks or straps.

In examples of the preceding aspects: (a) at least one said lower textile portion comprises one or more rigidized sections; and/or (b) the at least one said lower textile portion has a higher rigidity in an intermediate section thereof than at its ends.

In examples of the preceding aspects: (a) the positioning and stabilising structure comprises one or more sensors provided in or on the upper textile portion and/or one or more lower textile portions; (b) the positioning and stabilising structure comprises one or more actuators provided in or on the upper textile portion and/or one or more lower textile portions; (c) at least one sensor or at least one actuator is partly exposed to ambient at an exterior surface of the upper textile portion or the one or more lower textile portions; or is partly exposed at a patient-contacting surface of the upper textile portion or the one or more lower textile portions, so as to contact skin of the patient in use; (d) at least one sensor or at least one actuator is at least partly embedded between an outer layer and a patient-contacting layer of the upper textile portion or of one or more lower textile portions; (e) at least one sensor and/or at least one actuator comprises circuitry that is at least partially formed by one or more conductive threads, and/or one or more conductive ink traces; (f) the positioning and stabilising structure comprises one or more sensor-retaining structures for attachment of respective ones of the sensors and/or actuators; (g) the one or more sensor-retaining structures comprise one or more pockets to accommodate one or more respective sensors or actuators; (h) the positioning and stabilising structure comprises a wireless communications interface for transmitting data from the one or more sensors to one or more external computing devices, and/or for receiving data at the one or more actuators from the one or more external computing devices; and/or (i) the one or more sensors and/or one or more actuators comprise one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

In examples of the preceding aspects: (a) the first lower textile portion is integral with the upper textile portion; (b) the positioning and stabilising structure is in the form of a headband; (c) the headband comprises a first bifurcated section comprising a first part of the upper textile portion and further comprising the first lower textile portion, the first bifurcated section being located at a front of the headband; (d) the headband comprises a second bifurcated section comprising a second part of the upper textile portion and further comprising a second lower textile portion, the second bifurcated section being located at a rear of the headband; (e) the first lower textile portion is a seal retention band that is resiliently stretchable along at least part of its length, and is adapted to engage with an outer surface of the plenum chamber or the seal-forming structure to hold the seal-forming structure in the therapeutically effective position; (f) the seal retention band is more stretchable than the upper textile portion; (g) the seal retention band is received in a channel of the patient interface; (h) the channel is formed in an external surface of the plenum chamber; and/or (i) the seal retention band comprises a port for coupling the plenum chamber inlet port to an air circuit for supplying pressurised air to the patient.

In examples of the preceding aspects: (a) the positioning and stabilising structure comprises a pair of lower textile portions adapted to couple to each other, or to an intermediate structure, to provide said force; (b) the intermediate structure is a harness that, in use, engages with an external surface of the plenum chamber, or with an external surface of the seal-forming structure; (c) the intermediate structure comprises the plenum chamber and/or the seal-forming structure, or part thereof; (d) the plenum chamber and/or the seal-forming structure comprises one or more projections or recesses for coupling to resilient hooks located on the lower textile portions; (e) the lower textile portions couple to each other, and/or to the intermediate structure, by one or more resilient hooks or straps; (f) at least one said lower textile portion comprises one or more rigidized sections; and/or (g) the at least one said lower textile portion has a higher rigidity in an intermediate section thereof than at its ends.

In examples of the preceding aspects: (a) the patient interface comprises one or more sensors provided in or on the upper textile portion, and/or the at least one lower textile portion, and/or the plenum chamber, and/or the seal-forming structure; (b) the patient interface comprises one or more actuators provided in or on the upper textile portion, and/or the at least one lower textile portion, and/or the plenum chamber, and/or the seal-forming structure; (c) at least one sensor and/or at least one actuator is partly exposed to ambient at an exterior surface of the upper textile portion or the at least one lower textile portion; and/or is partly exposed at a patient-contacting surface of the upper textile portion or the at least one lower textile portion, so as to contact skin of the patient in use; (d) at least one sensor or at least one actuator is at least partly embedded between an outer layer and a patient-contacting layer of the upper textile portion or of the at least one lower textile portion; (e) at least one sensor and/or at least one actuator comprises circuitry that is at least partially formed by one or more conductive threads, and/or one or more conductive ink traces; (f) the patient interface comprises one or more sensor-retaining structures for attachment of respective sensors and/or actuators; (g) the one or more sensor-retaining structures comprise one or more pockets to accommodate one or more respective sensors or actuators; (h) the patient interface comprises a wireless communications interface for transmitting data from the one or more sensors to one or more external computing devices, and/or for receiving data at the one or more actuators from the one or more external computing devices; and/or (i) the one or more sensors and/or one or more actuators comprise one or more of: an accelerometer; a gyroscope; a humidity sensor; a temperature sensor; a microphone; a camera; a pulse oximeter; an EEG sensor; an EMG sensor; an EOG sensor; a touch sensor; a pressure sensor; a CO2 sensor; a vibration device; and an audio output device.

In examples of the preceding aspects: (a) the plenum chamber comprises a shell and has a shell inside surface and shell outside surface, and wherein the shell inside surface is arranged to be at said therapeutic pressure in use, and said shell outside surface is arranged to be at ambient pressure in use; (b) at least one lower textile portion engages with at least part of the shell outside surface to hold the seal-forming structure in the therapeutically effective position; (c) the channel is formed in the shell outside surface; (d) the shell is constructed from a hard plastic material; and/or (e) the shell is constructed from a transparent material.

One form of the present technology comprises a positioning and stabilising structure for a patient interface, comprising:

a headband formed at least partly from a textile material and having an upper textile portion movably connected to a first lower textile portion, the headband comprising one or more sensors provided in or on the upper textile portion and/or the first lower textile portion;

wherein the headband is wearable on a patient's head in a first configuration in which the first lower textile portion is adjacent to the upper textile portion, and a second configuration in which the first lower textile portion is separated from the upper textile portion and provides a force to hold a seal-forming structure of the patient interface in a therapeutically effective position on the patient's head.

In examples of the preceding aspects: (a) the positioning and stabilising structure comprises one or more actuators provided in or on the upper textile portion and/or the first lower textile portion; (b) at least one sensor and/or at least one actuator is partly exposed to ambient at an exterior surface of the upper textile portion or the first lower textile portion; and/or is partly exposed at a patient-contacting surface of the upper textile portion or the first lower textile portion, so as to contact skin of the patient in use; (c) at least one sensor and/or at least one actuator is at least partly embedded between an outer layer and a patient-contacting layer of the upper textile portion or of the first lower textile portion; (d) at least one sensor and/or at least one actuator comprises circuitry that is at least partially formed by one or more conductive threads, and/or one or more conductive ink traces; (e) the positioning and stabilising structure comprises one or more sensor-retaining structures for attachment of respective ones of the sensors and/or actuators; (f) the one or more sensor-retaining structures comprise one or more pockets to accommodate one or more respective sensors or actuators; (g) the positioning and stabilising structure comprises a wireless communications interface for transmitting data from the one or more sensors to one or more external computing devices, and/or for receiving data at the one or more actuators from the one or more external computing devices; and/or (h) the one or more sensors and/or one or more actuators comprise one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

In examples of the preceding aspects: (a) the first lower textile portion is integral with the upper textile portion; (b) the headband comprises a first bifurcated section comprising a first part of the upper textile portion and further comprising the first lower textile portion, the first bifurcated section being located at a front of the headband; (c) the headband comprises a second bifurcated section comprising a second part of the upper textile portion and further comprising a second lower textile portion, the second bifurcated section being located at a rear of the headband; (d) the first lower textile portion is a seal retention band that is resiliently stretchable along at least part of its length, and is adapted to engage with an outer surface of the patient interface to hold the seal-forming structure in the therapeutically effective position; (e) the seal retention band is more stretchable than the upper textile portion; (f) the seal retention band is adapted to be received in a channel of the patient interface; (g) the channel is formed in a plenum chamber of the patient interface; (h) the seal retention band comprises a port for coupling the seal-forming structure to an air circuit for supplying pressurised air to the patient; (i) the first lower textile portion comprises one or more rigidized sections; and/or (j) the first lower textile portion has a higher rigidity in an intermediate section thereof than at its ends.

One form of the present technology comprises a patient interface comprising:

a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient,

a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and

a positioning and stabilising structure according to any of the preceding aspects or examples.

In examples of the preceding aspects: (a) the patient interface further comprises one or more sensors positioned in or on an internal surface of the plenum chamber; and/or (b) the one or more sensors comprise one or more of: a pressure sensor; a humidity sensor; a temperature sensor; and a $CO_2$ sensor.

One form of the present technology comprises a system for diagnosis and/or monitoring of a respiratory disorder, comprising: a patient interface according to any of the preceding aspects or examples; and at least one computing device in communication with the patient interface to receive data from the one or more sensors of the patient interface.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

4 BRIEF DESCRIPTION OF THE DRAWINGS

The present technology is illustrated by way of example, and not by way of limitation, in the figures of the accompanying drawings, in which like reference numerals refer to similar elements including:

4.1 RESPIRATORY THERAPY SYSTEMS

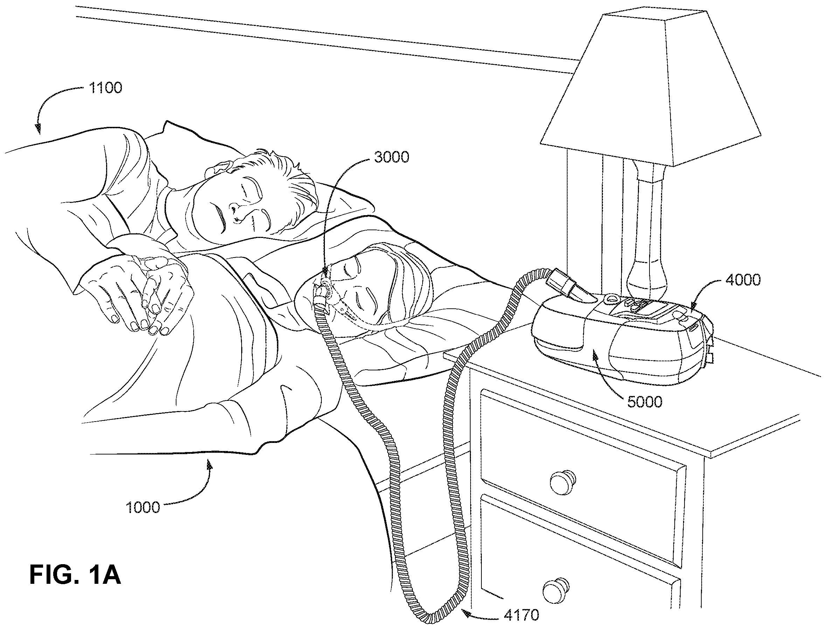

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

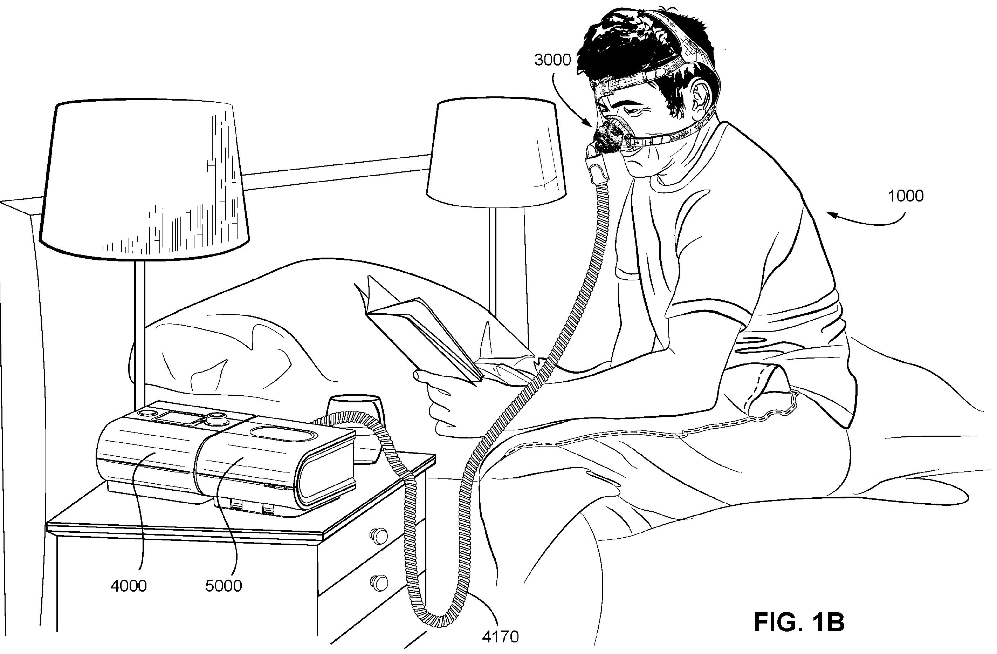

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

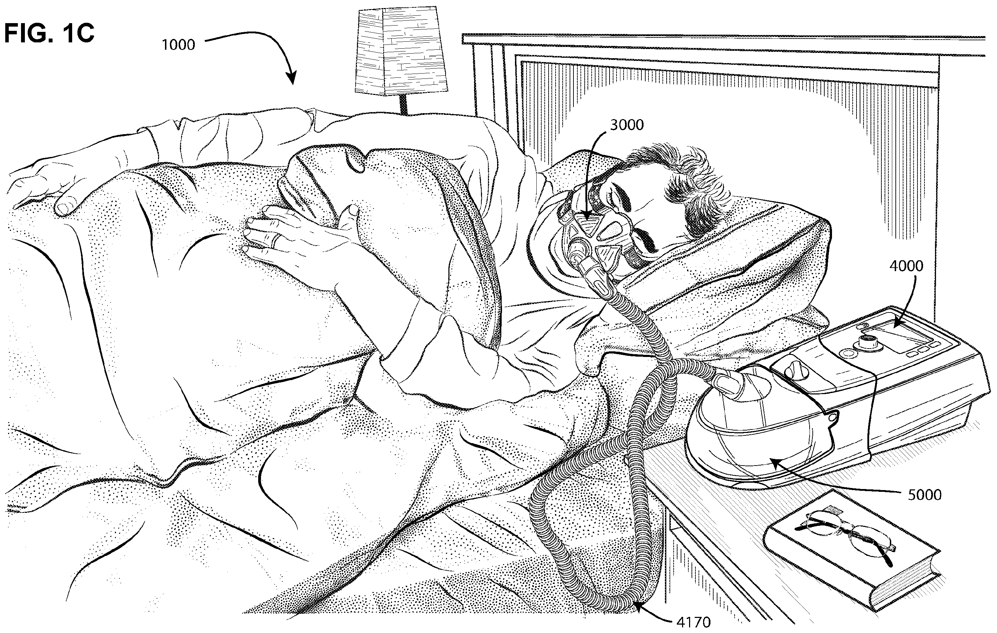

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

4.2 RESPIRATORY SYSTEM AND FACIAL ANATOMY

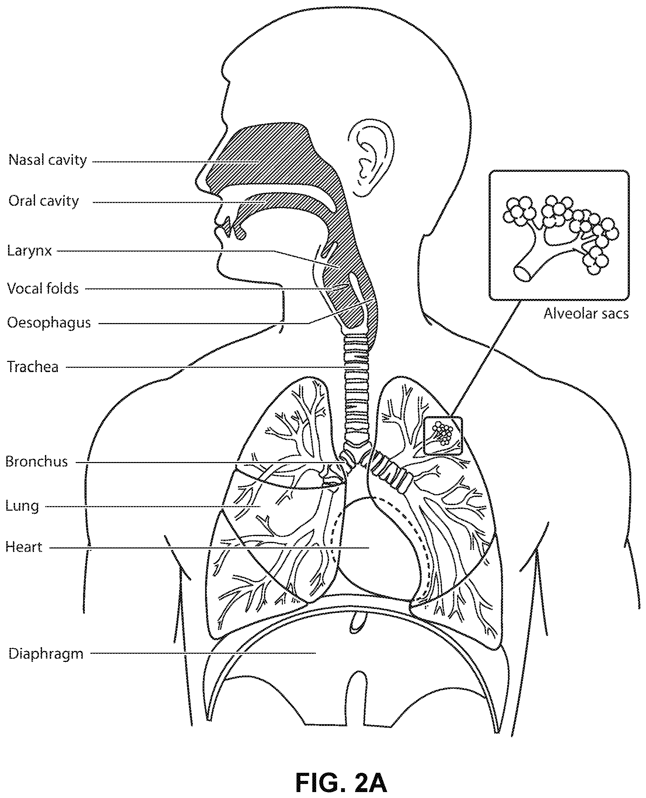

Fig. 2A shows an overview of a human respiratory system including the nasal and oral cavities, the larynx, vocal folds, oesophagus, trachea, bronchus, lung, alveolar sacs, heart and diaphragm.

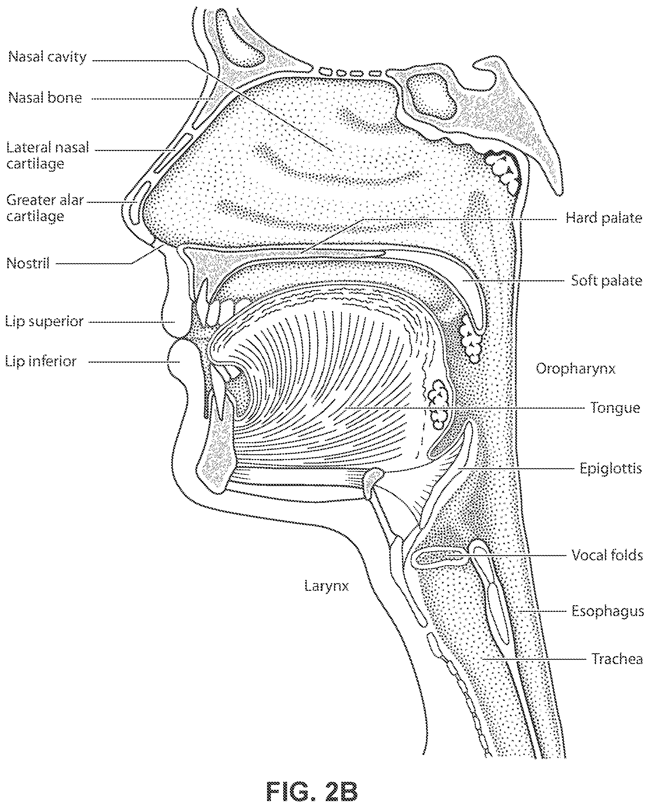

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

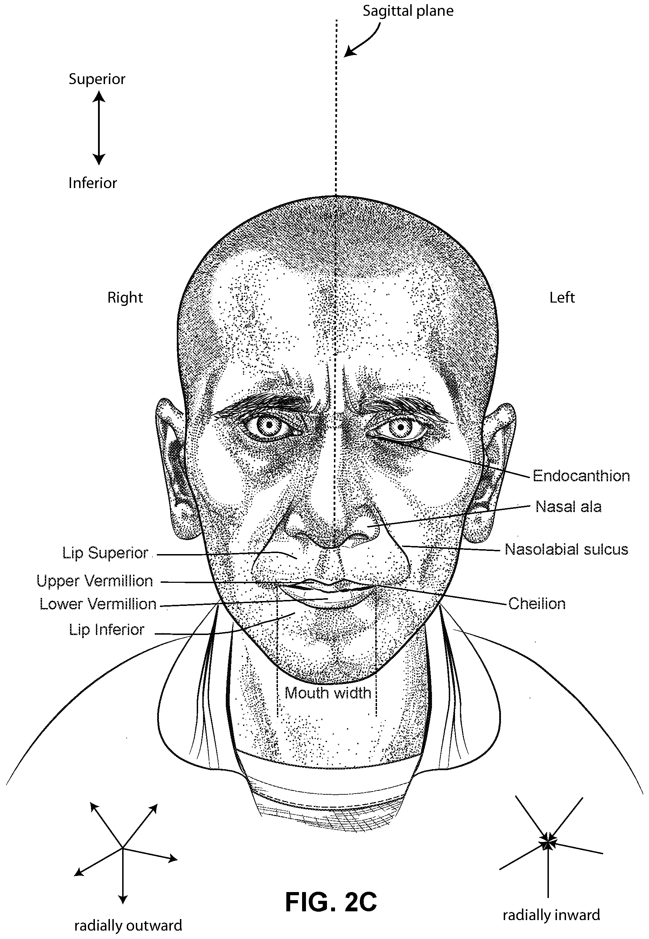

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

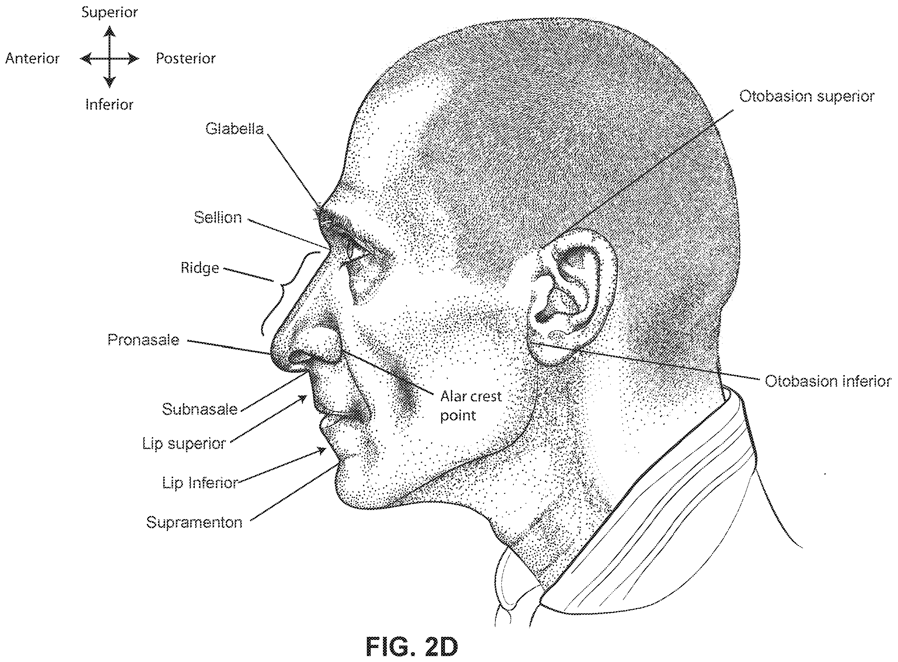

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

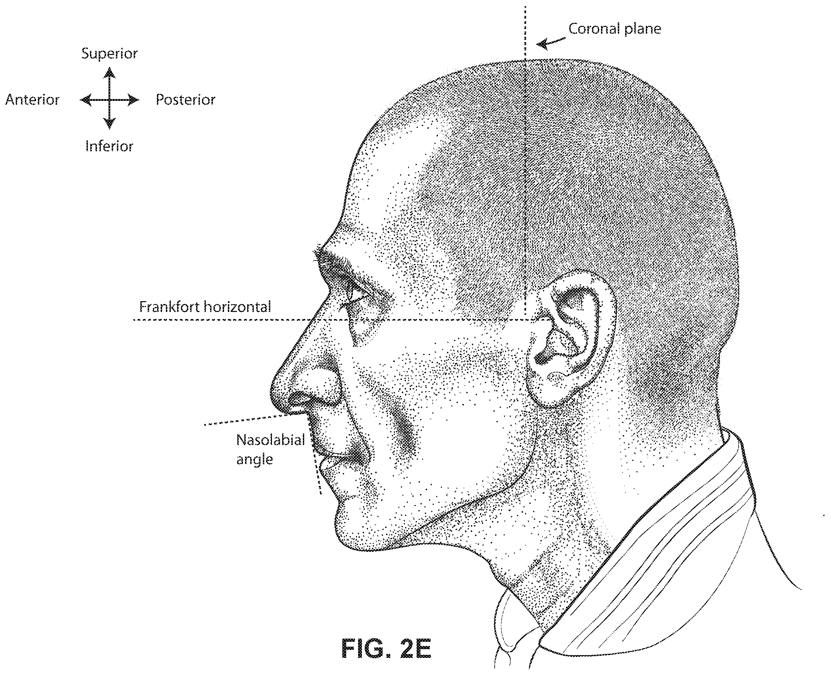

Fig. 2E is a further side view of a head. The approximate locations of the Frankfort horizontal and nasolabial angle are indicated. The coronal plane is also indicated.

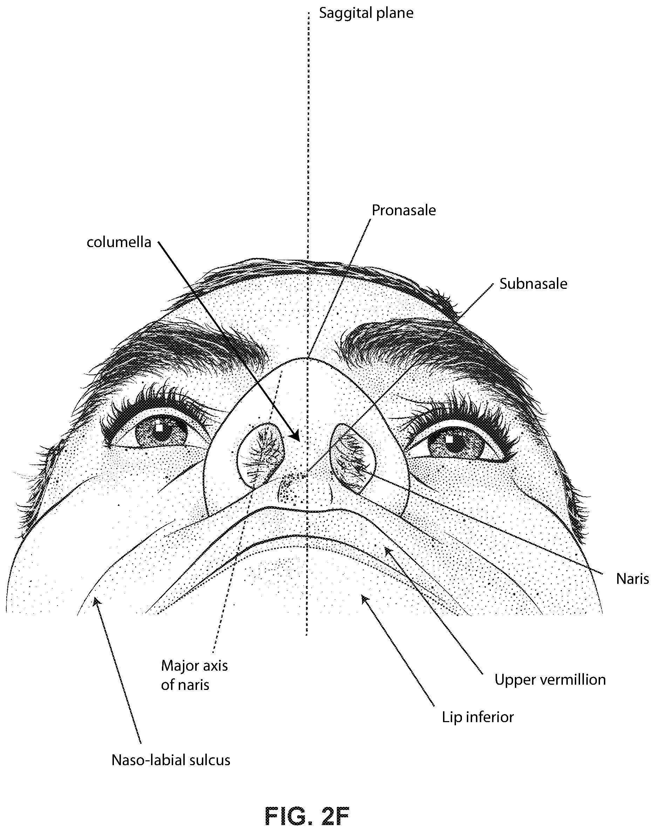

Fig. 2F shows a base view of a nose with several features identified including naso-labial sulcus, lip inferior, upper Vermilion, naris, subnasale, columella, pronasale, the major axis of a naris and the midsagittal plane.

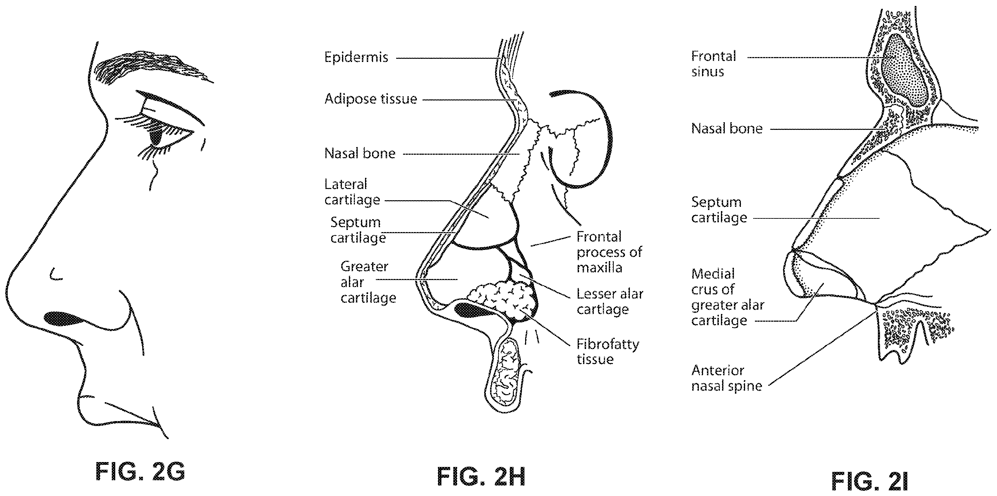

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

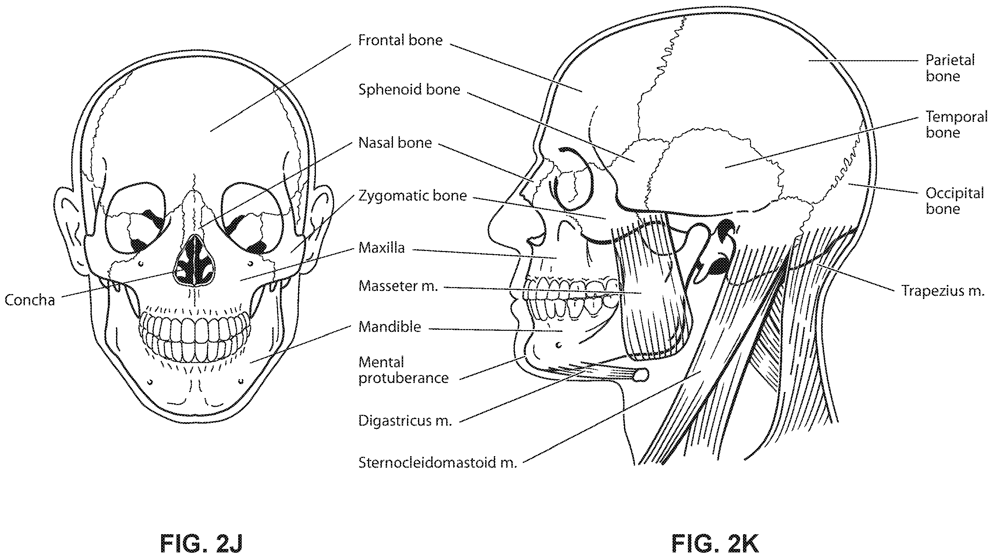

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

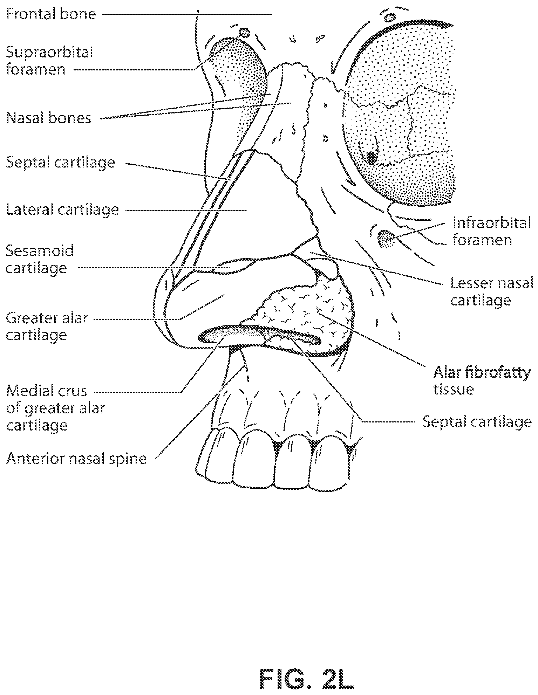

Fig. 2L shows an anterolateral view of a nose.

4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 3I, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

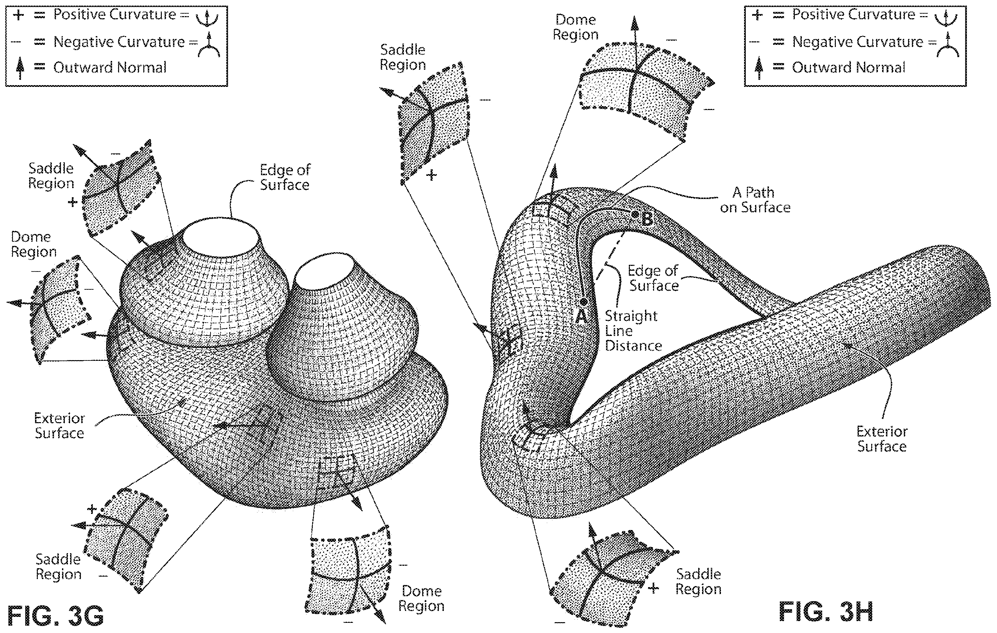

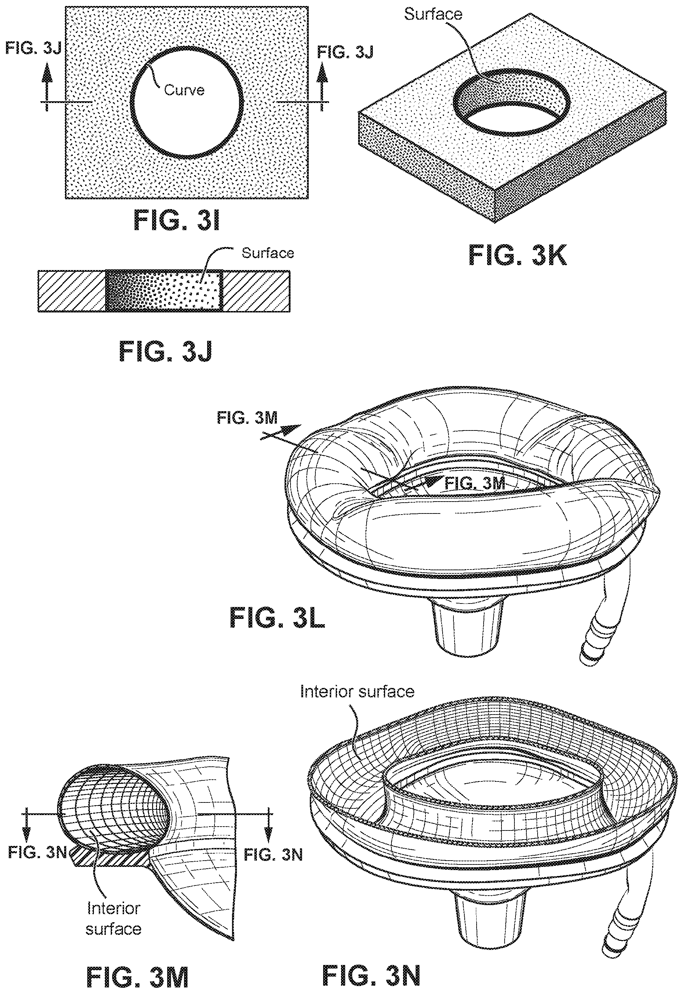

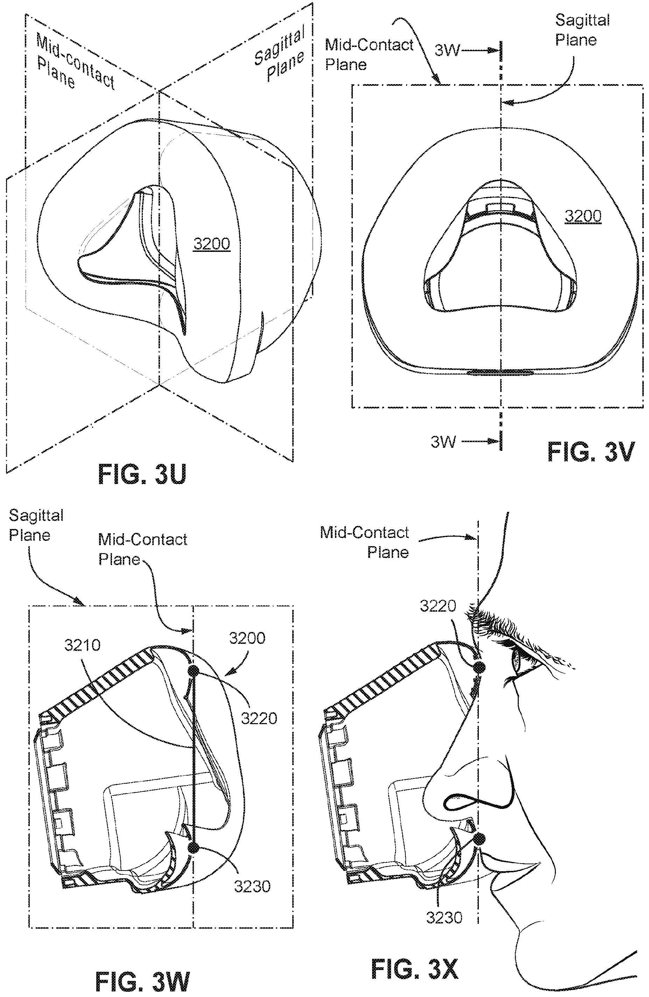

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

Fig. 4A is a front perspective view of a positioning and stabilising structure for a patient interface in accordance with one form of the present technology.

Fig. 4B is a side view of a patient interface in accordance with one form of the present technology, incorporating the positioning and stabilising structure of Fig. 4A, shown in an in-use position on a patient's head.

Fig. 4C is a front perspective view of the patient interface of Fig. 4B, shown in an in-use position on the patient's head.

Fig. 4D is a close-up view illustrating a support zone of the positioning and stabilising structure of Fig. 4A.

Fig. 4E is a side view of the positioning and stabilising structure of Fig. 4A worn by a patient in a first configuration.

Fig. 4F is a side view of the positioning and stabilising structure of Fig. 4A worn by a patient in a second configuration.

Fig. 5A is a side view of a patient interface in accordance with one form of the present technology, shown in an in-use position on a patient's head.

Fig. 5B is a schematic view of a mechanism for coupling a seal-forming structure to a positioning and stabilising structure for the patient interface of Fig. 5A.

Fig. 6 is a front perspective view of a patient interface in accordance with one form of the present technology, shown in an in-use position on a patient's head.

Fig. 7A is a side view of a patient interface in accordance with one form of the present technology, shown in an in-use position on a patient's head.

Fig. 7B is a schematic view of a mechanism for coupling a seal-forming structure to a positioning and stabilising structure for the patient interface of Fig. 7A.

Fig. 7C is a front perspective view of the patient interface of Fig. 7A, shown in an in-use position on a patient's head.

Fig. 8A is a front perspective view of a patient interface in accordance with one form of the present technology, shown in an in-use position on a patient's head.

Fig. 8B is a close-up side view of a plenum chamber of the patient interface of Fig. 8A.

Fig. 9A shows a positioning and stabilising structure for a patient interface in accordance with one form of the present technology, in a first in-use position on a patient's head.

Fig. 9B shows the positioning and stabilising structure of Fig. 9A, in a second in-use position on a patient's head as part of a patient interface.

Fig. 9C is a schematic cross-section through part of the positioning and stabilising structure of Fig. 9A.

4.4 RPT DEVICE

Fig. 10A shows an RPT device in accordance with one form of the present technology.

Fig. 10B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 10C is a schematic diagram of the electrical components of an RPT device in accordance with one form of the present technology.

Fig. 10D is a schematic diagram of the algorithms implemented in an RPT device in accordance with one form of the present technology.

Fig. 10E is a flow chart illustrating a method carried out by the therapy engine module of Fig. 10D in accordance with one form of the present technology.

## 4.5 HUMIDIFIER

Fig. 11A shows an isometric view of a humidifier in accordance with one form of the present technology.

Fig. 11B shows an isometric view of a humidifier in accordance with one form of the present technology, showing a humidifier reservoir 5110 removed from the humidifier reservoir dock 5130.

Fig. 11C shows a schematic of a humidifier in accordance with one form of the present technology.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.
In certain examples of the present technology, a supply of air at positive pressure is provided to the nasal passages of the patient via one or both nares.
In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying pressurised air to the patient 1000 via an air circuit 4170 and a patient interface 3000.

### 5.3 PATIENT INTERFACE

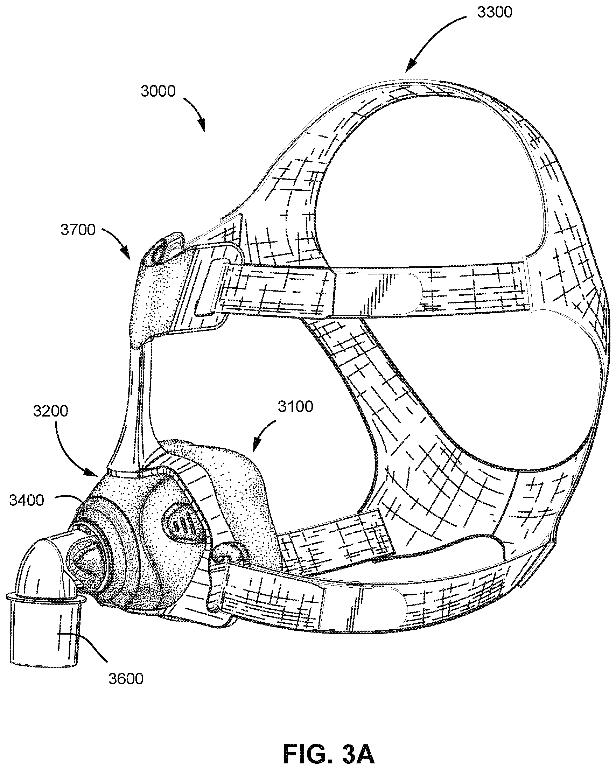

With reference to Fig. 3A, a non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

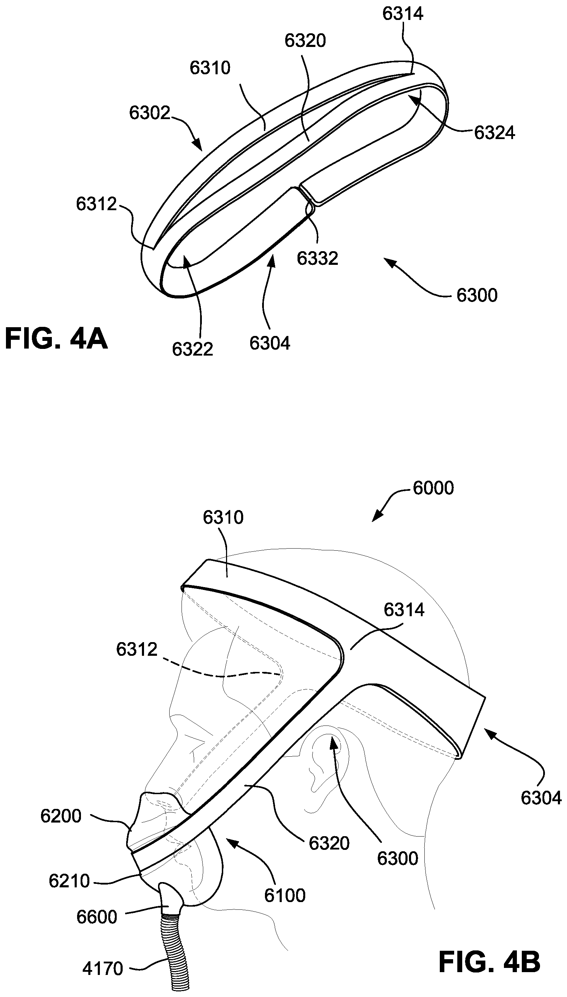

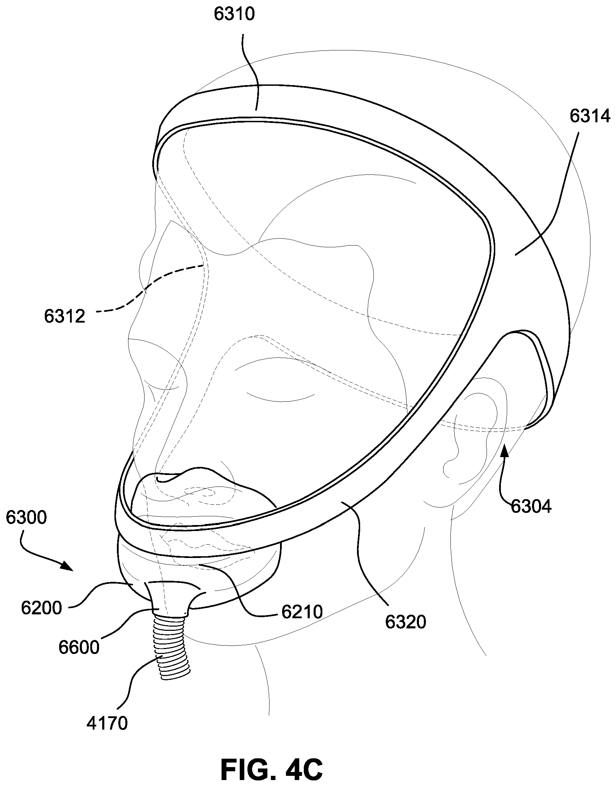

Other examples of non-invasive patient interfaces 6000, 7000, 8000, 9000, 10000 and 11000 are also illustrated in Figures 4A-4F, 5A-5C, 6, 7A-7C, 8A-8B, and 9A-9C, and will be described in further detail below.

If a patient interface is unable to comfortably deliver a minimum level of positive pressure to the airways, the patient interface may be unsuitable for respiratory pressure therapy.

The patient interface in accordance with one form of the present technology is constructed and arranged to be able to provide a supply of air at a positive pressure of at least 6 cmH$_2$O with respect to ambient.

The patient interface in accordance with one form of the present technology is constructed and arranged to be able to provide a supply of air at a positive pressure of at least 10 cmH$_2$O with respect to ambient.

The patient interface in accordance with one form of the present technology is constructed and arranged to be able to provide a supply of air at a positive pressure of at least 20 cmH$_2$O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.3 Positioning and stabilising structure

The seal-forming structure 3100, 6100, 7100, 8100, 9100, 10100, 11100 of the patient interface 3000, 6000, 7000, 8000, 9000, 10000, 11000 of the present technology may be held in sealing position in use by the positioning and stabilising structure 3300, 6300, 7300, 8300, 9300, 10300, 11300.

In one form the positioning and stabilising structure 3300, 6300, 7300, 8300, 9300, 10300, 11300 provides a retention force at least sufficient to overcome the effect of the positive pressure in the plenum chamber 3200, 6200, 7200, 8200, 10200, 11200 to lift off the face.

In one form the positioning and stabilising structure provides a retention force to overcome the effect of the gravitational force on the patient interface.

In one form the positioning and stabilising structure provides a retention force as a safety margin to overcome the potential effect of disrupting forces on the patient interface, such as from tube drag, or accidental interference with the patient interface.

In one form of the present technology, a positioning and stabilising structure is provided that is configured in a manner consistent with being worn by a patient while sleeping. In one example the positioning and stabilising structure has a low profile, or cross-sectional thickness, to reduce the perceived or actual bulk of the apparatus. In one example, the positioning and stabilising structure comprises at least one strap having a rectangular cross-section. In one example the positioning and stabilising structure comprises at least one flat strap.

In one form of the present technology, a positioning and stabilising structure is provided that is configured so as not to be too large and bulky to prevent the patient from lying in a supine sleeping position with a back region of the patient's head on a pillow.

In one form of the present technology, a positioning and stabilising structure is provided that is configured so as not to be too large and bulky to prevent the patient from lying in a side sleeping position with a side region of the patient's head on a pillow.

In one form of the present technology, a positioning and stabilising structure comprises a strap constructed from a laminate of a fabric patient-contacting layer, a foam inner layer and a fabric outer layer. In one form, the foam is porous to allow moisture, (e.g., sweat), to pass through the strap. Alternatively or in addition, the strap may comprise fiberfill (for example, polyester fiberfill), non-woven padding, foam padding, high density upholstery foam, compressed polyester, medium density polyurethane antimicrobial foam, high density polyurethane foam, dry fast open cell foam, or a combination thereof. Consequently, the strap is not too large and bulky to prevent the patient from lying in the side sleeping position. In addition, the strap is extensible and soft.

In certain forms of the present technology, a positioning and stabilising structure comprises a strap that is extensible, e.g. resiliently extensible. For example the strap may be configured in use to be in tension, and to direct a force to draw a seal-forming structure into sealing contact with a portion of a patient's face.

For example, referring to Fig. 4A-4F, a patient interface 6000 comprises a positioning and stabilising structure or headgear 6300 that comprises an upper textile portion 6310 comprising a circumferential band for fitting to the patient's head in use. A first lower textile portion 6320 is movably (e.g., articulable, hingedly, pivotably, etc.) connected to the upper textile portion 6310. For example, the first lower textile portion 6320 may be integral with the upper textile portion 6310. Alternatively, the first lower textile portion 6320 may be joined to the upper textile portion 6310, for example by stitching, ultrasonic welding, or other techniques. For example, respective ends of the first lower textile portion 6320 may be joined at respective seams 6322 and 6324, near bifurcation points 6312 and 6314, located at opposed lateral sides of the positioning and stabilising structure 6300. Since the textile portions 6310, 6320 are stretchable, the length of each portion when in use is equal to or greater than its corresponding resting length. Accordingly, creasing of the textile material of portions 6310, 6320 is avoided.

The positioning and stabilising structure 6300 is used to apply a force to a seal-forming structure 6100. The seal-forming structure 6100 may be a cushion mask having a plenum chamber 6200 and a connection port 6600 for connecting the plenum chamber 6200 to an air circuit 4170.

In this example, the positioning and stabilising structure 6300 is formed as a band having an anterior or front section 6302 and a posterior or back section 6304, where the front section 6302 is bifurcated (at points 6312, 6314) such that a first fork forms a first part of the upper textile portion 6310 and a second fork forms the first lower textile portion 6320. Accordingly, the upper textile portion 6310 forms a first band or strap that can encircle the patient's forehead in use, as shown in Fig. 4B, while the first lower textile portion 6320 forms a second band or strap that is downwardly stretchable to engage directly or indirectly with a seal-forming structure 6100 to provide a force to hold the seal-forming structure 6100 in a therapeutically effective position on the patient's head.

In some forms, the front section 6302 may have substantially the same width as the back section 6304. In other words, a width across both the first part of the upper textile portion 6310 and the first lower textile portion 6320 is substantially equivalent to the width of the back section 6304. This may allow the positioning and stabilising structure 6300 to have a substantially constant width of material at any point along the positioning and stabilising structure 6300.

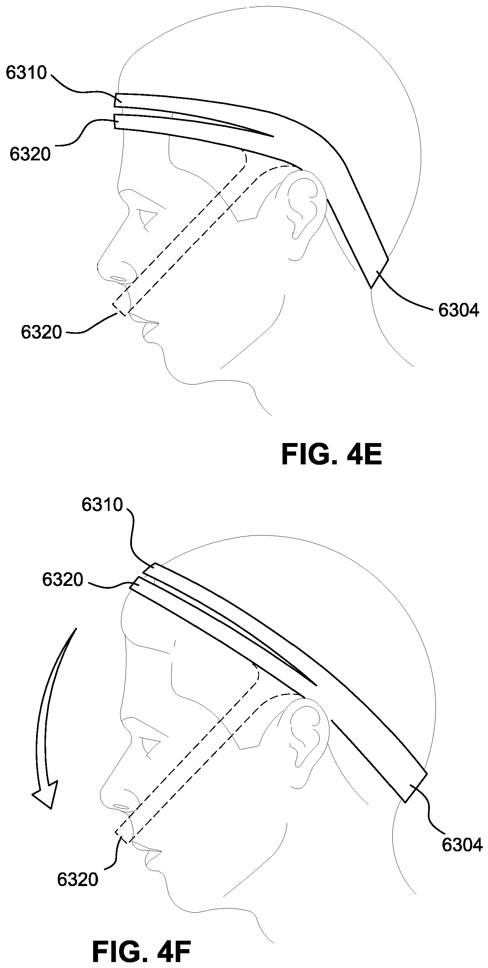

The configuration of Fig. 4A-4F allows the positioning and stabilising structure 6300 to be worn as a headband, with both textile portions 6310, 6320 encircling the patient's forehead, to enable the patient to become accustomed to the feel of the structure 6300 prior to commencing therapy. The upper textile portion 6310 may be configured to encircle the patient's forehead such that a front section (or an anterior portion) of the upper textile portion 6310 is configured to engage an anterior portion of the patient's head, and a back section (or a posterior portion) of the upper textile portion 6310 is configured to engage a posterior portion of the patient's head. Similarly, the lower textile portion 6320 may be configured to encircle the patient's forehead such that a back section (or a posterior portion) of the first lower textile portion 6320 is configured to engage the posterior portion of the patient's head. In this first position, the front section of the upper textile portion 6310 (i.e., forming a portion of front section 6302) and the lower textile portion 6320 may be positioned proximate (e.g., adjacent, close together, etc.) to one another on the patient's forehead. When in use, a front section (or an anterior portion) of the first lower textile portion 6320 is configured to move to a second position and overlie the cheek region of the patient's face, preferably the upper cheek region, and extend between the top of the patient's ear and the patient's eye. Further, prior to commencing therapy, the first lower textile portion 6320 can be readily pulled down (as depicted in the dotted outline in Fig. 4E and Fig. 4F), whereby it can engage with and provide a tensioning force to the plenum chamber 6200 (as depicted in Fig. 4B-4D), for example by nesting within an external groove or channel 6210 of the plenum chamber 6200. In the second position, the first lower textile portion 6320 may be positioned proximate to the patient's mouth (e.g., overlaying the patient's lip superior), while the upper textile portion 6310 may remain in substantially the same position (e.g., on the patient's forehead) so that the first lower textile portion 6320 is at least partially spaced from the upper textile portion. For example, the bifurcated portions of the front section 6302 may be spaced apart from each other (e.g., in order to contract the patient's head and avoid obstructing the patient's eyes). However, the front section 6302 and the rear section 6304 remain continuously formed in the second position, and the combined width of the spaced apart front section 6302 remains substantially equal to the width of the rear section 6304. Prior to commencing therapy, the positioning and stabilising structure 6300 may be worn without air circuit 4170 being connected and/or without the pressure generator 4140 of RPT device 4000 being switched on This enables the patient to become accustomed to wearing the patient interface 6000 without experiencing any discomfort from positive pressure provided by the pressure generator 4140. Accordingly, the patient is able to gradually transition to respiratory therapy, thus improving the likelihood of compliance once therapy actually starts.

The positioning and stabilising structure 6300 is thus readily transformable, by a simple pivoting movement, between the first portion or a non-therapy configuration (e.g., for monitoring and/or diagnostic purposes when sensors are provided in the positioning and stabilising structure 6300, as will be discussed below) and the second position or a therapy or pre-therapy configuration.

Some forms of the present technology may comprise a padding structure attached to or integrated with either or both of the upper textile portion 6310 and the lower textile portion 6320. The padding structure may comprise a layer of a soft foam or soft woven and/or knitted fabric or non-woven fabric that is integrated within, or affixed to, the upper and/or lower textile portions 6310, 6320. Alternatively, a padding structure may comprise one or more sleeves of such padding materials that are provided around an exterior of the upper textile portion 6310 and/or the lower textile portion 6320. The padding structure may provide improved comfort to the wearer.

In some forms of the present technology, the lower textile portion 6320 may be more stretchable than the upper textile portion 6310, thus enabling the lower textile portion 6320 to be more easily stretched downwardly, as illustrated in Figure 4F, to engage with plenum chamber 6200. For example, the stretch properties of portions 6310, 6320 may be modulated by adopting different types of knitting, such as warp knitting, weft knitting, or a combination of both. In one example, a narrow double-knitting bar warp knit may be used to form the lower textile portion 6320, or at least part thereof, to provide extra stretch and length relative to the upper textile portion 6310 for engagement with plenum chamber 6200.

Upper textile portion 6310 and lower textile portion 6320 may be made from the same fabric (or textile). The fabric may be nylon, polyester, polypropylene (PP), elastane, or a combination of any two or more thereof. The elasticity may be varied by changing the combination of the fabric materials (i.e. blend proportion), yarn count, yarn density, changing the yarn size, and/or changing steps and conditions of the process for manufacturing the fabric.

The fabric may comprise a core yarn that comprises a core and a covering. The core yarn may comprise polyurethane fibres and/or elastomeric fibres (such as rubber fibres and silicone fibres) to achieve the desired stretchability, while the covering may be made from nylon, polyester, and/or polypropylene. **In** addition, the filaments used for the covering may be textured continuous filaments to achieve better softness, durability, better thermal insulation, high permeability, and good moisture transport (i.e. moisture wicking).

A width of each of upper textile portion 6310 and lower textile portion 6320 may be less than 40 mm. In some forms, the width is less than 30 mm. The width may be more than 3 mm. The textile portions 6310 and 6320 may be of different widths. For example, upper textile portion 6310 may be wider to accommodate sensors and circuitry (e.g. greater than 10 mm and less than 30 mm, or greater than 15 mm and less than 25 mm), and lower textile portion 6320 may be narrower to avoid obstruction of vision (for example, less than 10 mm, or less than 15 mm).

The upper textile portion 6310 may be arranged to have a lower stretchability and greater thickness than the lower textile portion 6320 to ensure that the upper textile portion 6310 holds its shape, whereas the lower textile portion 6320 is arranged to be more easily stretched downwards (where the plenum chamber 6200 is retained for use). For example, the thickness of the fabric for upper textile portion 6310 may be in the range of about 0.30 mm to about 1.50 mm, and the thickness of the fabric for the lower textile portion 6320 may be in the range of about 0.20mm to about 1.00 mm.

The positioning and stabilising structure 6300 may be constructed by knitting a strip of material with the bifurcation points at 6312, 6314, and the ends of the strip of material may then be attached (at join 6332 as shown in Fig. 4A) to form the positioning and stabilising structure 6300.

In some embodiments, two separate parts may be knitted, and then attached together by any suitable means. For example, a first (upper) circumferential band may be joined to a second (lower) circumferential band by a join section or seam that extends partly around their respective circumferences, leaving at least one unjoined section (e.g., spanning between bifurcation points 6312 and 6314, or between seams 6322 and 6324) where the two bands are separable. The first and second circumferential bands may be formed from different textile materials and/or may have different degrees of stretchability. The join between the two parts may be formed by knitting, stitching, adhesives (including adhesive patches, which may be rigid or elastically deformable), ultrasonic bonding, heat sealing, or a combination of any two of these.

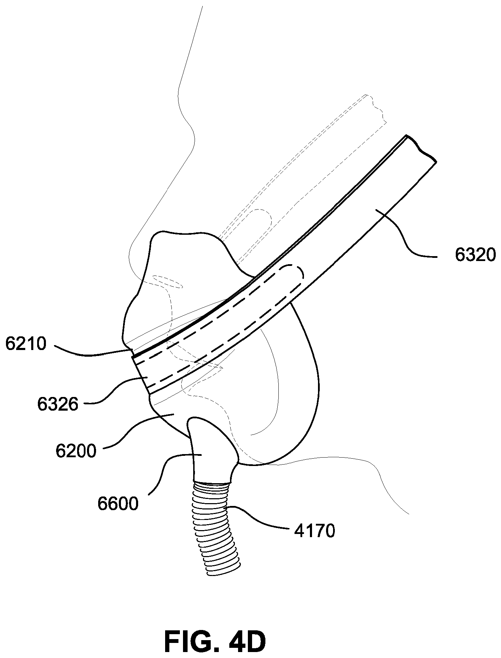

In some forms of the present technology, the degree of stretch or rigidity of the first lower textile portion 6320 may be varied along its length. For example, as depicted in Fig. 4D, an intermediate section 6326 of the first lower textile portion 6320 that is arranged to directly engage with the plenum chamber 6200 (e.g., within groove 6210 thereof) may be more rigid than the remainder of the first lower textile portion 6320 to provide greater support at the point of direct engagement. Other parts of the first lower textile portion 6320 may be rigidized so as to provide greater stability of the headgear 6300 in use, and/or to alter the direction of the tensioning force to prevent the first lower textile portion 6320 from riding up over the patient's cheekbone and covering the eye.

In some forms of the present technology, one or more rigidizers may be provided to selectively alter the rigidity of the first lower textile portion 6320. These may be attached to the first lower textile portion 6320, or inserted between layers thereof. For example, the intermediate section 6326 could comprise a rigidizer laminated to or embedded between layers of the lower textile portion 6320. Alternatively, thermoset yarns may be used to provide selective rigidification in the intermediate section 6326. The textile may also be rigidized at other parts of the lower textile portion 6320, such as along side sections that will contact the patient's face in use, for example using a coating, a laminate, a rigidized thread sewn into the textile, or any similar means.

In some forms, the positioning and stabilising structure may comprise a patient-contacting structure having one or more resilient straps extending therefrom to engage with a harness that retains the seal-forming structure in place.

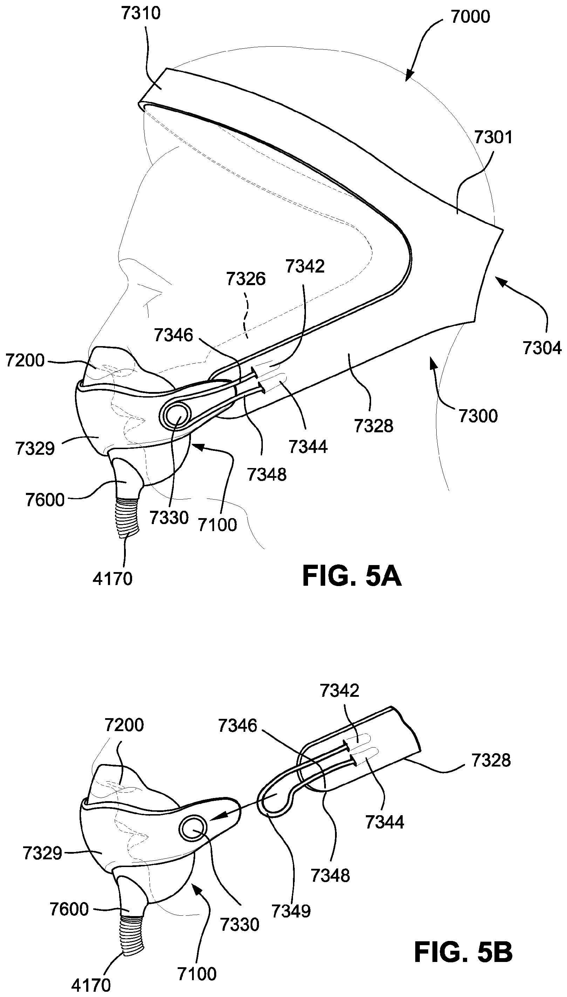

For example, as shown in Fig. 5A, a patient interface 7000 comprises a positioning and stabilising structure 7300, which in turn comprises a patient-contacting structure 7301 that cooperates with a harness 7329 to provide a tensioning force to seal-forming structure 7100 to maintain the seal-forming structure 7100 in a therapeutically effective position on the patient's head. The patient interface 7000 also comprises a plenum chamber 7200 having a connection port 7600 for connecting the patient interface 7000 to an air circuit 4170.

In one form of the present technology, the positioning and stabilising structure 7300 comprises an upper textile portion 7310, and two lower textile portions which are movably (e.g., articulable, hingedly, pivotably, etc.) connected to the upper textile portion 7310. Positioning and stabilising structure 7300 also comprises a posterior section 7304 that is configured to anchor against a posterior surface of a patient's neck. The upper textile portion 7310 is adapted to encircle the patient's head in use, in a region that is superior to an otobasion superior of the patient's head.

The lower textile portions may comprise a first arm 7326 and a second arm 7328. Each arm may have a hook, e.g. hook 7349 extending from second arm 7328 (Fig. 5B), to engage with a corresponding catch 7330 located on the harness 7329. For example, the hook 7349 may be formed from a length of an elastic yarn material, opposed ends 7346, 7348 of which are located within respective channels 7342 and 7344 on the second arm 7328. By forming the hook 7349 from an elastic material, it may be readily extended to engage with the catch 7330, while also being able to retract back into the channels 7342, 7344 when the patient interface 7000 is not in use and the seal-forming structure 7100 does not need to be attached. Although not specifically shown in Fig. 5A and 5B, it will be appreciated that the same structure (or a similar structure) may be provided on the first arm 7326, to engage with a further catch on the opposite side of the harness 7329, such that the two hook-catch pairs cooperate to retain the harness 7329, and thus the seal-forming structure 7100, in place on the patient's head.

Hook 7349 is shown in Fig. 5A and Fig. 5B as extending from an external (non-patient contacting) surface of the second arm 7328. It will be appreciated that in some forms of the present technology, hook 7349 may instead extend from channels located on the internal (patient-contacting) surface of the second arm 7328, though it will generally be desirable to use the external placement as shown in Fig. 5A and Fig. 5B, for greater patient comfort.

The first arm 7326 and second arm 7328 may each vary in rigidity relative to the upper textile portion 7310 and the posterior portion 7304 of the patient-contacting structure 7301 along at least part of their lengths. For example, each of the first and second arms 7326, 7328, in at least an intermediate section thereof, may be more rigid than the upper textile portion 7310 and the posterior portion 7304. By providing increased rigidity in at least the intermediate sections of the two arms 7326, 7328, the positioning and stabilising structure 7300 provides increased support to the seal-forming structure 7100, making it less prone to movement during respiratory therapy, and providing a better dynamic seal. Further, similarly to the rigidification mentioned above in relation to positioning and stabilising structure 6300, selectively increasing the rigidity in the arms 7326, 7328 assists to direct the tensioning force vector away from the patient's eyes during use. In some embodiments, the arms 7326, 7328 may be formed using thermoset yarns to provide the desired rigidity.

In one form, one or more rigidizers may be provided in arms 7326, 7328. The rigidizers may be semi-rigid. In other words, the rigidizers may be more rigid than the textile material used to form the arms 7326, 7328 and/or the upper textile portion 7310, but not completely rigid. In this way, they are capable of providing structure to the arms 7326, 7328, but are flexible so that they are capable of being bent. A patient and/or medical professional may adjust or bend the rigidizers in order to provide tailored support for an individual patient. The rigidizers also may begin semi-rigid (in other words, the rigidizers may be semi-rigid at the beginning or initially), and may become rigid after a period of time. For example, a medical professional may adjust the shape of the rigidizers so that the positioning and stabilising structure 7300 is suited for an individual patient's face. Then the rigidizers may be treated (e.g., heat treated) so that they are set in their shape. In other words, the rigidity of the rigidizers is capable of changing. Consequently, the rigidity of the arms 7326, 7328 also changes and may be selectively increased to provide tailored support for the individual patient (or patient).

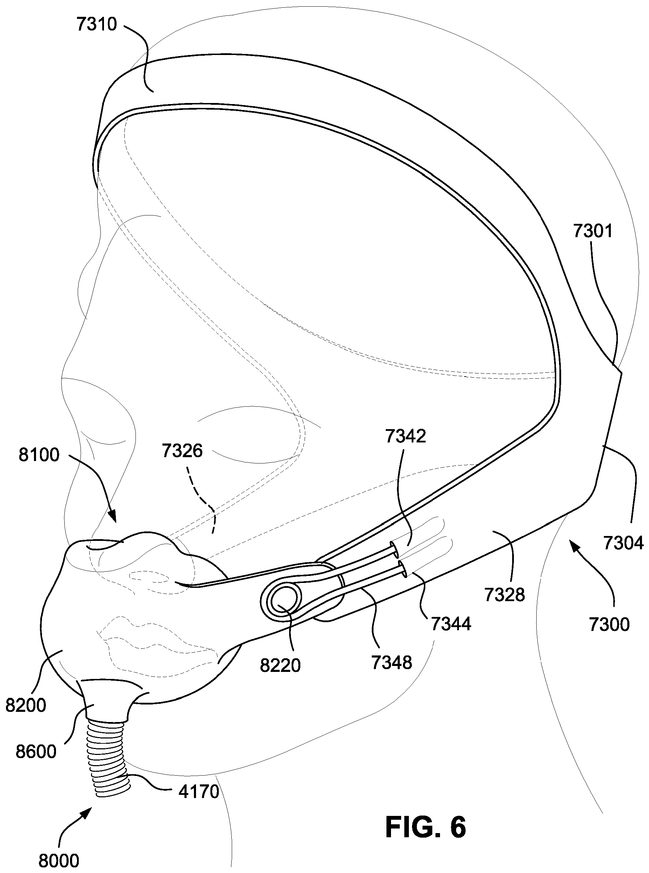

In one form of the present technology, as shown in Fig. 6, a patient interface 8000 comprises a seal-forming structure 8100 that comprises one or more catches 8220 that can engage with corresponding hooks on a positioning and stabilising structure 7300 to retain the seal-forming structure 8100 in a therapeutically effective position on a patient's head. For example, the patient-contacting structure 7301 of the positioning and stabilising structure 7300 may cooperate with such a seal-forming structure 8100 to form the patient interface 8000, which also comprises a plenum chamber 8200, and a connection port 8600 for connection to an air circuit 4170. Accordingly, the patient-contacting structure 7301 may either be used with an intermediate structure such as a harness as shown in Fig. 5A and Fig. 5B in order to retain the seal-forming structure, or may engage directly with the seal-forming structure (where the seal-forming structure comprises a suitable engagement mechanism such as catch 8220 of structure 8100). The harness-type configuration is advantageous in that it may be used with many types of existing seal-forming structure 7100, whereas the configuration in Fig. 6 is advantageous in that, by virtue of the direct engagement with the seal-forming structure 8100, a more stable support for the seal-forming structure 8100 may be provided.

In some embodiments, a plurality of catches 8220 may be provided on the seal-forming structure 8100, or another structure connected thereto such as plenum chamber 8200, at a plurality of different positions, such that the tension in the positioning and stabilising structure 7300 may be adjusted by attaching the hooks 7349 to the catches 8220 at the different positions. Likewise, a plurality of catches 7330 may be provided at a plurality of different positions on the harness 7329 of Fig. 5A and 5B for this purpose.

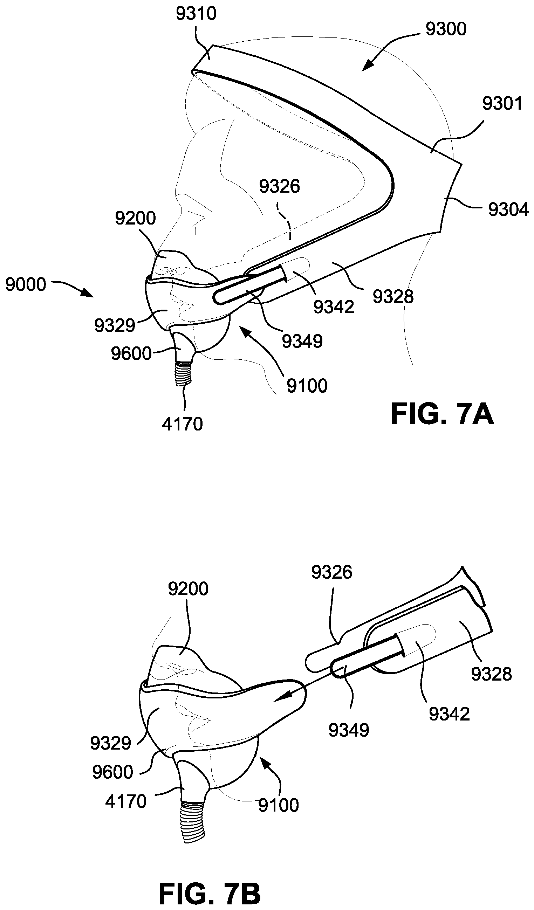

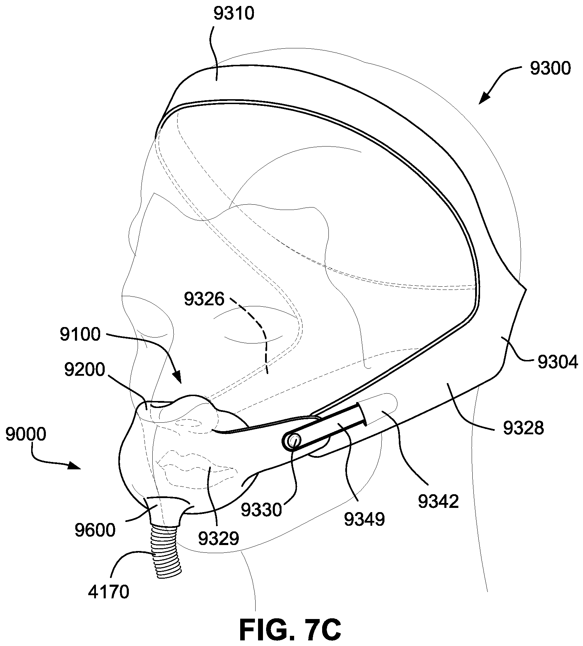

In a further form of the present technology, as shown in Fig. 7A-7C, a patient interface 9000 comprises a positioning and stabilising structure 9300, which in turn comprises a patient-contacting structure 9301 that cooperates with a harness 9329 to provide a tensioning force to a seal-forming structure 9100 to maintain the seal-forming structure 9100 in a therapeutically effective position on the patient's head. The patient interface 9000 also comprises a plenum chamber 9200 having a connection port 9600 for connecting the patient interface 9000 to an air circuit 4170. The positioning and stabilising structure 9300 of patient interface 9000 is similar to positioning and stabilising structure 7300 of the patient interface 7000, but with at least a different engagement mechanism for connecting the harness 9329 to the patient-contacting structure 9301.

In one form of the present technology, the positioning and stabilising structure 9300 comprises an upper textile portion 9310, and lower textile portions which are movably (e.g., articulable, hingedly, pivotably, etc.) connected to (for example, being integral with) and extend from the upper textile portion 9310. Positioning and stabilising structure 9300 also comprises a posterior section 9304 that is configured to anchor against posterior surfaces of a patient's neck or occipital bone. The upper textile portion 9310 is adapted to encircle the patient's head in use, in a region superior to an otobasion superior of the patient's head.

The lower textile portions may comprise a first arm 9326 and a second arm 9328. Each arm may have a hook, e.g. hook 9349 extending from second arm 9328 (Fig. 7B), to engage with a corresponding catch (not shown) located on the harness 9329. For example, the hook 9349 may be an elastic strap that is housed in a channel 9342 on an external (non-patient contacting) surface of the second arm 9328, and which can be extended from the channel 9342 to engage with the catch on the harness 9329. When the strap 9349 is released, it retracts at least partly back into the channel 9342.

The strap 9349 may attach to harness 9329 by a hook-and-loop arrangement. For example, a first patch carrying hooks (or loops) on its surface may be provided on the strap 9349, and a second patch carrying loops (or hooks) on its surface may be provided on the harness 9329, such that the patient can attach the strap 9349 to the harness 9329 by contacting the first patch with the second patch. Further, the tension may be adjusted by changing the relative positions of the first and second patches when in contact. The hook-and-loop arrangement may comprise a hook component that comprises nanoscale protrusions, and a loop component that comprises nanofiber-pile fabric. One such hook-and-loop system is sold by Teijin Limited under the trade mark FASTENANO. **In** another possible arrangement, the strap 9349 may comprise an array of spikes or like protrusions that can embed into the material of harness 9329 for attaching the harness.

Although not specifically shown in Fig. 7A-7C, it will be appreciated that the same structure (or a similar structure) may be provided on the first arm 9326, to engage with a further catch on the opposite side of the harness 9329, such that the two hook-catch pairs cooperate to retain the harness 9329, and thus the seal-forming structure 9100, in place on the patient's head.

The first arm 9326 and second arm 9328 may each vary in rigidity relative to the first resilient textile portion 9310 and the posterior portion 9304 of the patient-contacting structure 9301 along at least part of their lengths. For example, each of the first and second arms 9326, 9328, in at least an intermediate section thereof, may be more rigid than the first resilient textile portion 9310 and the posterior portion 9304. By providing increased rigidity in at least the intermediate sections of the two arms 9326, 9328, the positioning and stabilising structure 9300 provides increased support to the seal-forming structure 9100, making it less prone to movement during respiratory therapy. Advantageously, the arms 9326, 9328 may be formed using thermoset yarns to provide the desired rigidity. Alternatively, or in addition, similar rigidification mechanism as discussed above may be employed, such as the provision of rigidizers that are attached to or embedded between layers of the arms 9326, 9328.

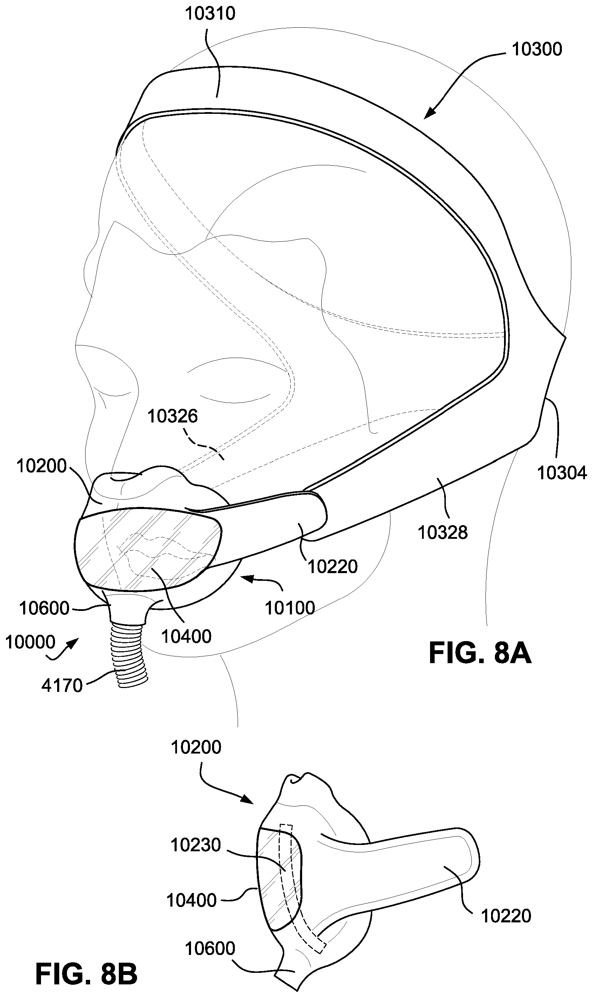

In a yet further form of the present technology, illustrated in Fig. 8A and 8B, a patient interface 10000 comprises a positioning and stabilising structure 10300 and a plenum chamber 10200 having a connection port 10600 for connecting the patient interface 10000 to an air circuit 4170. The positioning and stabilising structure 10300 is constructed and arranged to provide a force to maintain a seal-forming structure 10100 of the patient interface on a patient's head in a therapeutically effective position.

In one form of the present technology, the positioning and stabilising structure 10300 comprises an upper textile portion 10310, and lower textile portions which are movably (e.g., articulable, hingedly, pivotably, etc.) connected (for example, integral with) and extend from the first resilient textile portion 10310. Positioning and stabilising structure 10300 also comprises a posterior section 10304 that is configured to anchor against posterior surfaces of a patient's neck. The first resilient textile portion 10310 is adapted to encircle the patient's head in use, for example a region superior to an otobasion superior of the patient's head.

The lower textile portions may comprise a first arm 10326 and a second arm 10328. Each of the first arm 10326 and the second arm 10328 may be resiliently coupled, in use, with the plenum chamber 10200 to maintain the seal-forming structure 10100 in place. For example, plenum chamber 10200 may have a first wing (not shown) and a second wing 10220 extending therefrom, each being adapted to couple to respective arms 10326, 10328. For example, each wing may carry a part of a fastener such as a button, clip or the like, that is adapted to mate with a corresponding part on a respective arm 10326 or 10328 to fasten the wing to the arm 10326, 10328. Alternatively, a hook-and-loop arrangement (such as a nanofiberbased hook-and-loop arrangement) may be used to attach the first and second wings to the arms 10326, 10328 in analogous fashion to that described above with reference to positioning and stabilising structures 7300 and 9300. Further, the wings may carry spikes or like structures that can penetrate into the arms 10326 and 10328 for attachment. If the wings are formed from a textile material, or have a textile outer layer, spikes or similar structures may be carried on external or internal (patientfacing) surfaces of the arms 10326 and 10328 to embed into fibres of the textile material of the wings for attachment.

The wings 10220 may be formed from various materials, such as textile, polymers, elastomers, or a combination thereof.

The first arm 10326 and second arm 10328 may each vary in rigidity relative to the upper textile portion 10310 and the posterior section 10304 along at least part of their lengths. For example, each of the first and second arms 10326, 10328, in at least an intermediate section thereof, may be more rigid than the upper textile portion 10310 and the posterior section 10304. The arms 10326, 10328 may be formed using thermoset yarns to provide the desired rigidity, and/or using one or more rigidizers provided in or on the arms 10326, 10328. Respective ends of the arms 10326, 10328 that connect to the wings 10220 may be relatively more elastic than the intermediate sections, such that, when connected to the wings, they provide the requisite tensioning force to maintain seal-forming structure 10100 in place.

In some forms of the present technology, a humidity exchanger 10230 may be provided inside plenum chamber 10200. Humidity exchanger 10230 is a passive component positioned in the flow path to absorb moisture and heat from the exhalation flow and impart it to the inflow. One benefit of including a humidity exchanger 10230 in the plenum chamber is that it may reduce or eliminate the need for active humidification (such as by humidifier 5000). The humidity exchanger 10230 reduces the potential for dry mouth, which may be particularly important for patients who are prone to mouth-breathing and who may suffer discomfort as a result. It will be appreciated that like humidity exchangers may be provided in plenum chambers of any of the other forms of the technology described herein.

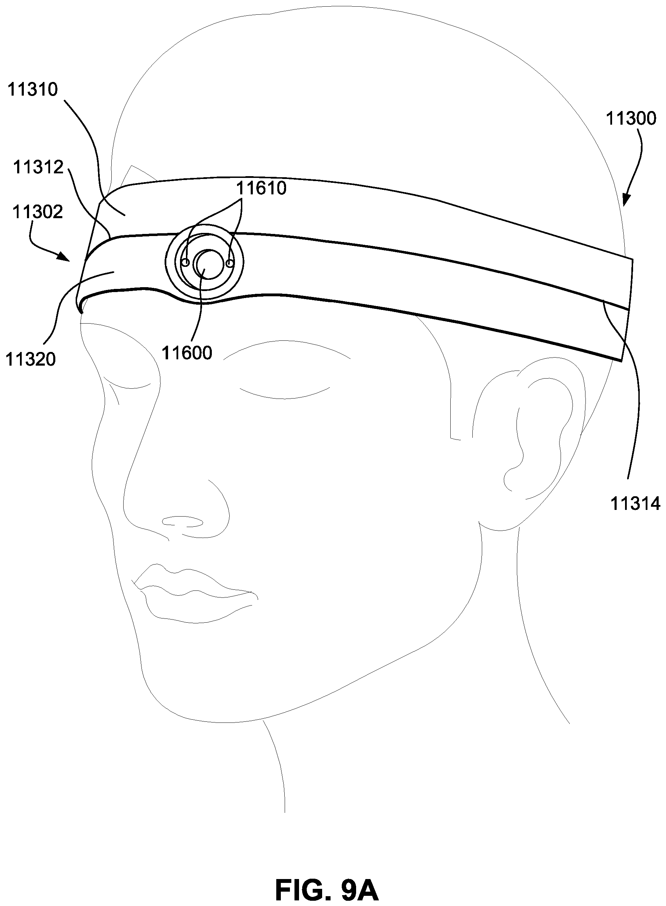

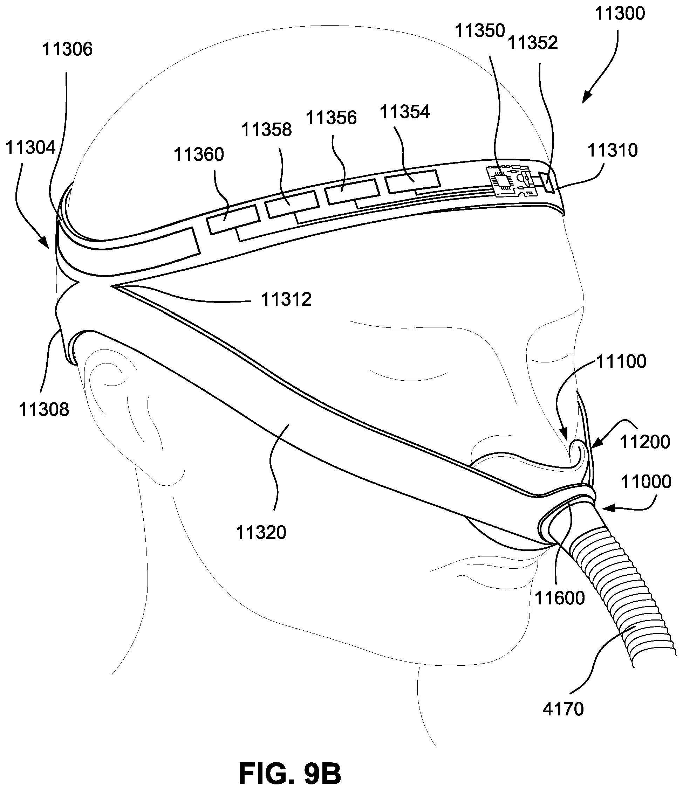

In one form of the present technology, as shown in Fig. 9A and 9B, a positioning and stabilising structure 11300 of a patient interface 11000 may have a connection port 11600 integrated therein, to facilitate transformation from a first position or a non-therapeutic configuration where the positioning and stabilising structure is worn as a headband, as in Fig. 9A, to a second position or a therapeutic (or pre-therapeutic) configuration, as in Fig. 9B, where the positioning and stabilising structure 11300 is connectable to an air circuit 4170 which is in communication with a plenum chamber 11200, via the connection port 11600.

A number of different forms of connection between air circuit 4170 and connection port 11600 are possible, provided that a substantially air-tight seal is formed to substantially prevent pressure leakage during therapy. For example, the port 11600 may comprise magnetic elements 11610 located at an interior surface thereof for connection to corresponding magnetic elements located on a connector at one end of air circuit 4170. In another example, the connector of the air circuit may attach to connection port 11600 by a snap-fit (such as an annular snap-fit or cantilever snap-fit, either of which may be rigid-to-rigid or rigid-to-resilient) or friction fit.

As shown in Fig. 9A, the positioning and stabilising structure 11300 comprises an upper textile portion 11310 comprising a resilient circumferential band for fitting to the patient's head in use. A first lower textile portion 11320 is movably (e.g., articulable, hingedly, pivotably, etc.) connected to (for example, integral with), and extends from, the upper textile portion 11310. The positioning and stabilising structure 11300 is used to apply a force to a seal-forming structure 11100, as shown in Fig. 9B. The seal-forming structure 11100 may be a nasal mask having a plenum chamber 11200. Other types of mask, such as full face masks and oro-nasal masks, may also be used with positioning and stabilising structure 11300 as part of a patient interface.

In this example, the positioning and stabilising structure 11300 is formed as a band having a front (anterior) section 11302 and a back (posterior) section 11304, where the front section 11302 has a first bifurcated section (spanning between bifurcation points 11312, 11314) such that a first fork forms a first part of the upper textile portion 11310 and a second fork forms the first lower textile portion 11320. Accordingly, the upper textile portion 11310 forms a first band or strap that can encircle the patient's forehead in use, as shown in Fig. 9A, while the first lower textile portion 11320 forms a second band or strap that is downwardly stretchable to engage directly or indirectly with seal-forming structure 11100 to provide a force to hold the seal-forming structure 11100 in a therapeutically effective position on the patient's head.

In some forms of the present technology, the posterior section 11304 may also comprise a bifurcated section, comprising a first posterior portion 11306 that is a second part of the upper textile portion 11310 and a second posterior portion 11308 that is a second lower textile portion, as shown in Fig. 9B. The bifurcation of the posterior section 11304 enables a greater degree of support, since tension can be provided at spaced locations at the back of the patient's head or occiput or adjacent to the occipital bone, and also provides for a greater degree of adjustability such that the patient may better position the positioning and stabilising structure 11300 for greater comfort. It will be appreciated that the posterior sections 6304, 7304, 9304, 10304 of any of the other forms of the present technology disclosed herein may also be bifurcated in like manner to posterior section 11304 of the positioning and stabilising structure 11300 of Fig. 9B.

In some forms, the posterior section 11304 may be similar to the previously described front or anterior sections (e.g., front section 6302). For example, the posterior section 11304 may include a width that combines the measured widths of the first posterior portion 11306 and the second posterior portion 11308. This combined width may be substantially similar to the combined width of the first lower textile portion 11320 and the first part of the upper textile portion 11310 (e.g., which together form the front section 11302).

The posterior section 11304 may also be movable between a first position and a second position. Similar to above, the posterior section 11304 may be in the first position when the first posterior portion 11306 and the second posterior portion 11308 are positioned proximate to one another. The posterior section 11304 may be in the second position when the first posterior portion 11306 and the second posterior portion 11308 are spaced apart from one another.

In certain forms (see e.g., Fig. 9A), the posterior section 11304 may be in the first position when the first and second posterior portions 11306, 11308 are positioned in an inferior portion on the patient's head (e.g., overlaying the occipital bone, proximate to the patient's next, etc.). Alternatively, the first and second posterior portions 11306, 11308 may be disposed slightly higher on the patient's head so that at least one overlays the parietal bone.

In certain forms (see e.g., Fig. 9B), the posterior section 11304 may be in the second position when the first and second posterior portions 11306, 11308 are spaced apart from each other. When the entire positioning and stabilising structure 11300 is in the second position, the front section 11304 and the posterior section 11306 may combine to form an X-shape when viewed from the side of the patient's head.

In certain forms, the first posterior portion 11306 may be moved (e.g., in the superior direction to overlay the parietal bone) relative to the second posterior portion 11308 from the first position to the second position. In other forms, the second posterior portion 11308 may be moved (e.g., in the inferior direction) relative to the first posterior portion 11306 from the first position to the second position. In still other forms, both the first posterior portion 11306 and the second posterior portion 11308 may be moved (e.g., in the superior direction to overlay the parietal bone) relative to the second posterior portion 11308 away from one another from the first position to the second position.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

**5.3.3.1 Sensor and actuator arrangements for patient interfaces**

In some forms of the present technology, a patient interface may have one or more sensors and/or actuators provided therein, for measurement of the patient's physiological and sleep data. One or more sensors and one or more actuators may respectively be embedded within the patient interface, for example between textile layers of headgear of the patient interface, or may be attached to internal and/or external surfaces of the headgear or other components of the patient interface. For example, one or more sensors and/or actuators may be integrated in a positioning and stabilising structure, and/or in another component such as a seal-forming structure or plenum chamber.

In some forms, the headgear may comprise one or more leads, cables, or other electrically conductive elements extending therefrom and being in electrical communication with one or more of the sensors or actuators. Each such electrically conductive element may comprise a terminal that can contact skin of the wearer of the headgear to provide one or more suitable signal grounding points on the face or head of the wearer, such as behind the ear, or below the eye socket. This may be useful for implementation of an EEG, EMG, or EOG system within the headgear.

Sensors embedded in the patient interface can help collect sleep-related data and physiological indicators such as vital data; this can be used to determine the improvement in sleep and health by comparing data before and after start of therapy. This data can be processed and the patient informed of how the therapy is improving sleep. For example, a patient may wear a positioning and stabilising structure 11300 with integrated sensors as a headband, as shown in Fig. 9A, before commencing therapy, and physiological and sleep data may be recorded while the patient is sleeping (and during the day, in the case of physiological data). After commencing therapy, with the positioning and stabilising structure 11300 in the therapeutic configuration shown in Fig. 9B, further physiological and sleep data may be recorded, and compared to the data recorded before commencement of therapy. The physiological and sleep data may be communicated to external computing devices such as a smartphone of the patient, and/or a monitoring server that is operated by or accessible to a clinician or other healthcare provider.

With a smoother acclimatisation due to the patient being able to wear the positioning and stabilising structure 11300 as a headband, and data-based feedback being obtained on how the therapy is actually helping (for example, via an application executing on the patient's smartphone), patient compliance is more likely. The data collected may also be useful in determining population-level sleep and/or physiological characteristics of one or more cohorts of patients undergoing respiratory therapy, thereby potentially enabling better customisation of therapy to patients falling within particular categories, or otherwise enabling optimisation of operation of an RPT device 4000.

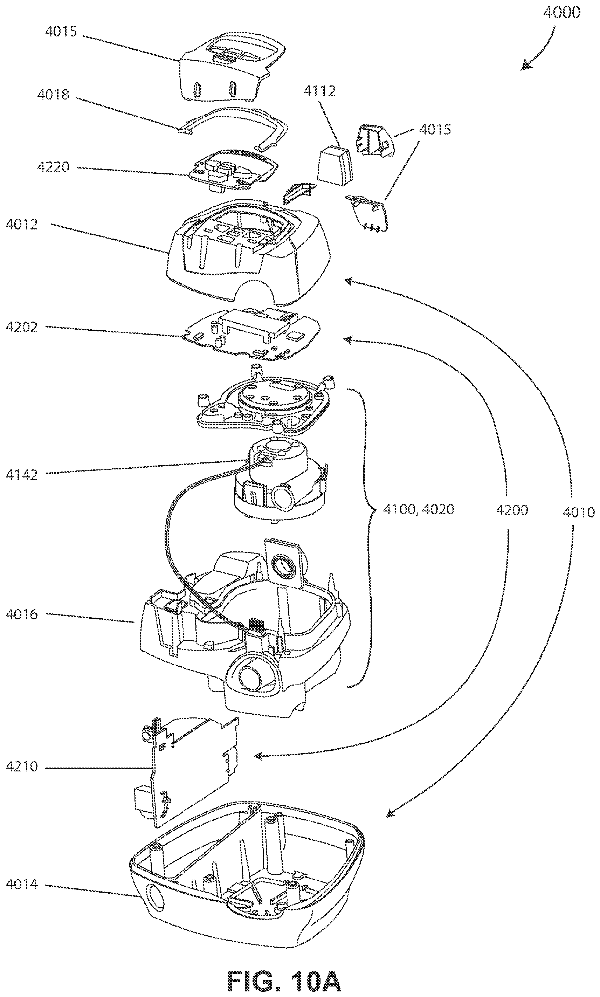

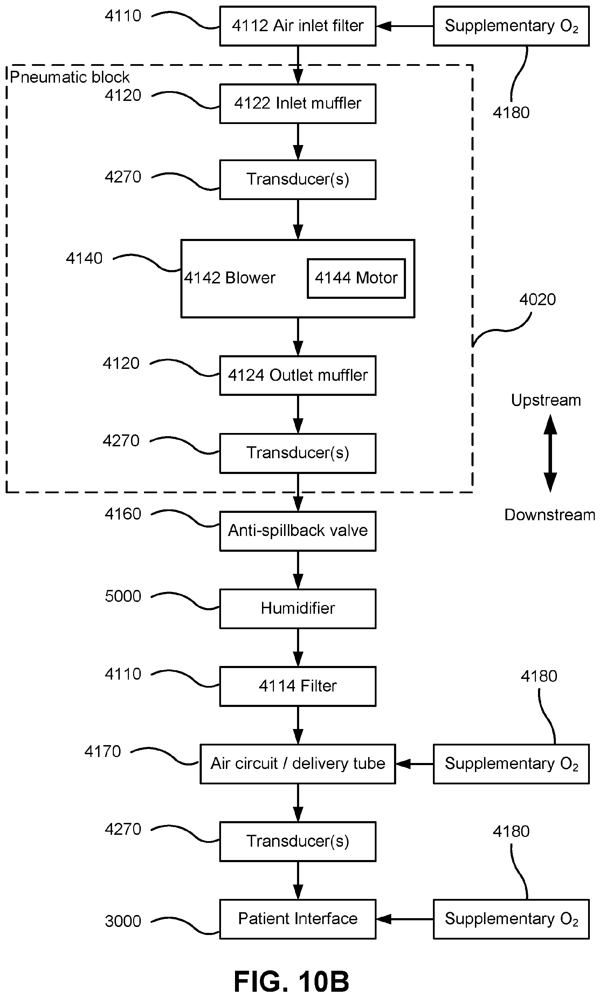

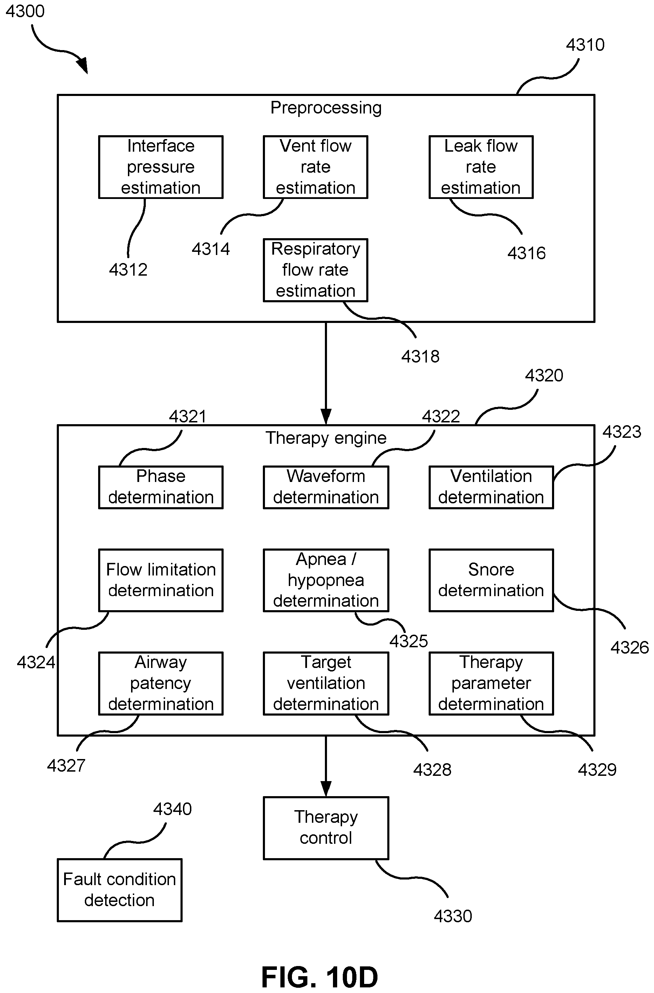

In some forms of the present technology, measurement of functional parameters at the patient side using sensors integrated in and/or attached to the mask may provide improved, active feedback-based control of an RPT device 4000 to which the patient interface is connected, for example, improved feedback control of a pressure generator 4140 of the RPT device 4000 (Fig. 10B).

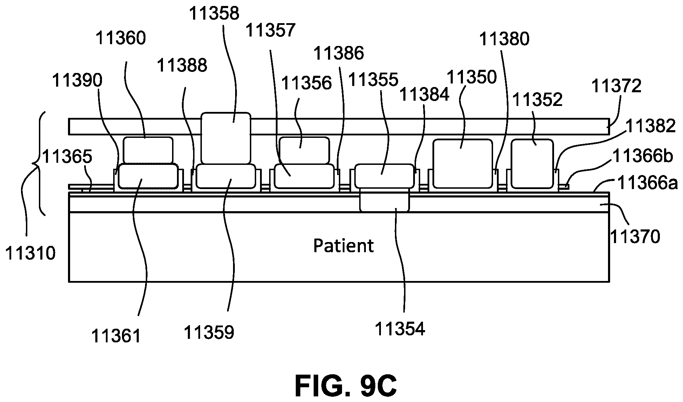

For example, referring to Fig. 9B and 9C, upper textile portion 11310 of positioning and stabilising structure 11300 may have a plurality of electronic modules (actuators and/or sensors) 11354, 11356, 11358 and 11360 integrated therein. A processor module 11350 may also be integrated in the positioning and stabilising structure 11300, typically also in the upper textile portion 11310, though it will be appreciated that the processor module 11350 may be located elsewhere within the positioning and stabilising structure 11300. The processor module 11350 may have an integrated transceiver for transmitting data to, and receiving data from, external computing devices. A battery 11352 is also included to power the various electronic components (sensors/actuators 11354-11360 and processor module 11350) of the positioning and stabilising structure 11300.

As shown in Fig. 9C, the sensors and associated electronics may be integrated at least partly between fabric layers of the upper textile portion 11310. For example, various sensor/actuator modules and/or associated circuitry, the processor module 11350, and the battery module 11352 may lie between an inner, patient-contacting, fabric layer 11370, and an outer, non-patient-contacting, fabric layer 11372.

The sensor and/or actuator modules integrated in the positioning and stabilising structure 11300 may be in electrical communication with processor 11350 and battery 11352 via a bus 11365, for example. The bus 11365 may be provided between two insulating layers 11366 that provide electrical insulation and that also prevent moisture ingress, for example from perspiration absorbed by inner fabric layer 11370. The insulating layers 11366 may be non-conductive polymer or elastomer films, for example, though it will be appreciated that other electrically insulating materials may also be used.

In some forms of the present technology, a thermally insulating layer may be provided between at least some of the electronic components of the positioning and stabilising structure 11300, noting that those components will tend to generate heat during use. Accordingly, a thermally insulating layer helps to improve patient comfort. For example, layer 11366 that is closest to the patient-contacting inner layer 11370 may be thermally insulating as well as electrically insulating, or an additional thermally insulating layer may be interposed between electrically insulating layer 11366 and inner layer 11370. In some examples, inner layer 11370 may itself be thermally insulating.

In some forms of the present technology, the sensor and/or actuator modules 11354-11360 and their associated circuitry, and other modules including the processor 11350 and battery 11352, may be received within sensor-retaining structures 11380-11390 that are affixed to insulating layer 11366 and/or inner fabric layer 11370. Each sensor-retaining structure 11380-11390 is in electrical communication with bus 11365, for example, and may contain electrical contacts to electrically connect circuitry of (or associated with) the sensor modules to bus 11365, and thus also to battery 11352 and processor 11350. In some examples, communication between modules 11350-11360 and bus 11365 may be via conductive ink traces, and/or conductive threads that are woven into or otherwise integrated with fabric layers 11370 and/or 11372. In some embodiments, electrical contacts and/or circuit traces may be contained only in outer layer 11372, so as not to be affected by perspiration from the patient during use.

In some examples, the modules 11350-11360 may be detachable from the sensor-retaining structures 11380-11390, such that particular modules may be switched out for other modules with different functionality, or to replace modules that have ceased to function or are at the end of their lifecycle. For example, the modules 11350-11360 (and/or the circuitry modules 11355, 11357, 11359 and 11361 to which they are electrically coupled, if applicable) may releasably attach to the sensor-retaining structures 11380-11390. To this end, an external surface of a module may form a friction fit with an internal surface of a wall of a sensor-retaining structure 11380-11390, or may form a snap fit, such as an annular snap fit or cantilever snap fit, with the wall or other internal or external part of the sensor-retaining structure. In some embodiments, a non-mechanical coupling, such as magnetic coupling, may be used to retain the modules 11350-11360 in respective sensor-retaining structures 11380-11390.

In some forms of the present technology, sensor-retaining structures 11380-11390 may comprise pockets formed in the upper textile portion 11310 (for example, by making incisions in outer layer 11372 or inner layer 11370), into which modules 11350-11360 (or their associated circuitry) are insertable to electrically couple with the bus 11365.

Battery module 11352 may comprise a rechargeable battery. The battery may be recharged by connecting it to an external power source, for example via a micro-USB or USB-C port of the battery module 11352 (the port being exposed via outer fabric layer 11372, for example), or by inductive charging. In some embodiments, the battery 11352 may be a disposable battery, for example disposed within a pocket 11382 of the upper textile portion 11310, and may be removable by the patient for replacement with a fresh battery.

In some forms of the present technology, one or more sensor modules and/or actuator modules may be enclosed entirely between the fabric layers 11370, 11372, such that no part of the one or more sensor modules is exposed. For example, an actuator module 11360 may be coupled to associated circuitry 11361 that is received in a sensor-retaining structure 11390. Both the actuator module 11360 and circuitry 11361 lie entirely between the fabric layers 11370, 11372. In another example, a sensor module 11356 and associated circuitry 11357 may lie entirely between fabric layers 11370, 11372. One example of a sensor module 11356 that may be fully embedded is an accelerometer or gyroscope.

In some forms of the present technology, a sensor module or actuator module may be at least partly exposed. For example, a humidity sensor 11358 coupled to circuitry 11359 may be at least partly exposed to ambient through the outer fabric layer 11372 to measure humidity of the patient's environment. To this end, outer fabric layer 11372 may comprise an aperture through which a surface of the humidity sensor 11358 may be exposed. In another example, a sensor 11354 coupled to circuitry 11355 may have a surface thereof exposed through the inner fabric layer 11370 (e.g., through an aperture formed therein), such that the sensor surface can contact the skin of the patient when the positioning and stabilising structure 11300 is worn by the patient. The sensor 11354 may be a pulse oximeter, for example.

Although the electronic components are described above as being modular in construction, and in at least some cases able to be switched out for other components, in some forms of the present technology, one or more electronic components (such as sensors or actuators) may be woven or otherwise integrated into the material of the upper textile portion 11310, for example into the outer fabric layer 11372 or the inner fabric layer 11370, and/or into another part of the positioning and stabilising structure 11300, such as lower textile portion 11320, and/or either or both of the posterior portions 11306, 11308. This may enable distribution of a sensor over a larger area for more informative and/or accurate measurements to be made.

In some forms of the present technology, a sensor may comprise a touch sensor, such as a capacitive or resistive sensor or tactile switch, and may have associated circuitry that enables the sensor to function as a "pause" button. For example, the touch sensor may be incorporated into an exposed region of the positioning and stabilising structure 11300, or of the seal-forming structure 11100. In one example, a touch sensor 11358 may be positioned on the upper textile portion 11310 in the manner shown in Fig. 9C. Touch sensor 11358 may communicate with processor/transceiver 11350 as previously described, such that signals recorded by touch sensor 11358 and circuitry 11359 may be transmitted by processor/transceiver 11350 to an external device, such as pressure generator 4140 of RPT device 4000.

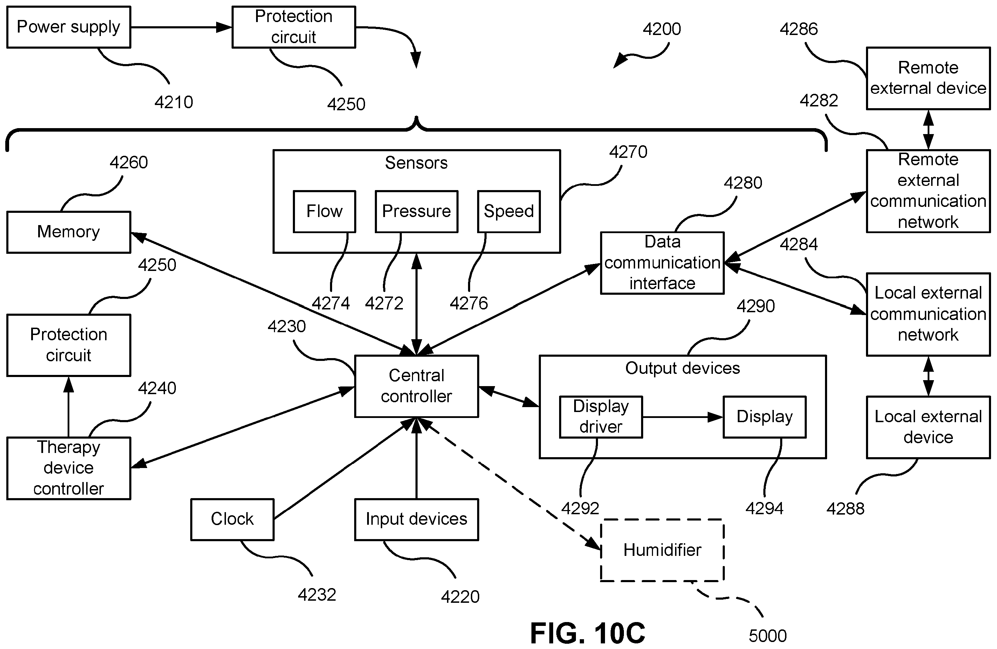

For example, if the patient has patient interface 11000 in place and wishes to talk, or wakes up in the middle of the night and is feeling uncomfortable due to the positive pressure in plenum chamber 11200, the patient may activate the "pause" sensor 11358 by a light continuous touch. Circuitry 11359 may detect this touch and transmit a pause signal to pressure generator 4140 (for example, via data communication interface 4280, Fig. 10C) to cause the flow to immediately be reduced to a very low value (for example, just enough to avoid a feeling of suffocation). When the pause sensor 11358 is released (or re-set or re-activated), this is detected by circuitry 11359 and a further signal sent to pressure generator 4140 to cause a ramp up algorithm implemented by RPT device 4000 to be reset.

Some forms of the present technology may comprise one or more sensors for determining sleeping position and movements of a patient prior to, and/or during, respiratory therapy. In some forms, the determined sleeping position and movements may be used to regulate the operation of pressure generator 4140, and/or to provide a sensory stimulus to the patient to cause them to change position. For example, if a number of apnea and/or hypopnea events above a certain threshold, and/or a decrease in blood oxygenation, is detected by the one or more sensors (whether or not pressure generator 4140 is operational at the time), this may be indicative of back sleeping. One or more actuators may receive an activation signal based on the detection, and the activation signal may cause the one or more actuators to generate a vibration or other tactile stimulus to irritate the patient sufficiently to cause them to switch to another sleeping position.

For example, positioning and stabilising structure 11300 or seal-forming structure 11100 may incorporate an accelerometer and/or gyroscope. The accelerometer and/or gyroscope may be fully enclosed between fabric layers 11370 and 11372 of the upper textile portion 11310, for example as shown at 11360 in Fig. 9C. Both the accelerometer and gyroscope are in communication with processor/transceiver 11350 such that data recorded by them may be transmitted to RPT device 4000 to regulate the operation of pressure generator 4140.

Measurements recorded by the accelerometer may be used to determine the patient's sleeping position and to adjust therapy accordingly. When it is detected that the patient is lying on their back, the therapy pressure can be ramped up slowly by pressure generator 4140 to prevent sleep apnea events. When side sleeping is detected, the therapy pressure can be lowered. When an upright (e.g. reading before sleep, with mask on) position is detected, the flow and pressure could be just sufficient to avoid a feeling of suffocation.

Measurements recorded by the gyroscope may be used to determine movements of the patient and to adjust therapy accordingly. When a lot of movement is detected, indicating that the patient is likely awake, the therapy pressure can be kept low enough to avoid a feeling of suffocation. When the movements subside, the therapy pressure can be very slowly ramped up to avoid discomfort.

In some forms of the present technology, accelerometer and/or gyroscope measurements may be used to determine a sleep stage of the patient, and to turn pressure generator 4140 on or off accordingly. For example, it may be difficult for a patient to fall asleep if therapy commences while they are still awake. Accordingly, pressure generator 4140 may remain in an "off" or paused state if the accelerometer and/or gyroscope measurements are indicative of an awake or light sleep stage, and then switched on (typically, with a gentle ramp-up) once measurements indicate that the patient is in a deep sleep stage. Conversely, if therapy has already commenced and it is detected that the patient has switched from deep to light sleep, where therapy may rouse the patient, the pressure generator 4140 may be paused until the patient is in deep sleep again, for example.

In some forms of the present technology, a pulse oximeter incorporated in the positioning and stabilising structure 11300 may be used to assess sleep health. For example, a pulse oximeter 11354 and associated circuitry 11355 may be incorporated in the upper textile portion 11310 of positioning and stabilising structure 11300, as shown in Fig. 9C. The pulse oximeter 11354 is exposed through an aperture of inner fabric layer 11370 such that it can contact the skin of the patient's forehead. Measurements recorded by pulse oximeter 11354 may be used to determine blood oxygen saturation level and heart rate during the period that the patient interface 11000 is worn, and this data may be transmitted to RPT device 4000, or an external computing device such as a smartphone, other mobile computing device, or laptop or desktop computing system of the patient. The time series data may be consolidated to provide feedback to the patient on their health levels, and recommendations for follow-up (for example, by a clinician).

In one example, an Apnea Hypopnea Index (AHI), which is a measure that clinicians use to classify the severity of sleep apnea, may be determined based on sensor measurements. Computation of AHI may use a combination of data from different sensors, e.g. blood oxygen level and heart rate (for example, measured by a PPG sensor), and chest movement (for example, measured by an accelerometer and/or gyroscope). The AHI value may be used to determine when an "apnea" occurs.

Accordingly, by tracking the AHI over time, a clinician will be able to tell if the patient has sleep apnea, and provide details of how severe it is. Further, by analysis of the AHI data together with other sensor data, the clinician may be able to not only correlate the frequency of apneas with particular sleeping positions (e.g. sleeping on back or sleeping on side), but also to adjust the CPAP therapy to the specific needs of the patient. For example, the amount of mouth breathing may be detected using temperature and/or humidity sensors located inside the plenum chamber of the patient interface, and a nasal or full-face mask prescribed accordingly. Additionally, pressure generator 4140 settings that will produce a flow rate most suitable for the patient may be recommended based on the sensor measurements. For example, a clinician may prescribe higher pressure settings (or equivalently, higher flow rates) for patients with a high detected rate of apnea or hypopnea events. The prescribed flow rate may also depend on the anatomical structure of the patient, for example if the patient has a more collapsible upper airway.

In some forms of the present technology, an EEG sensor may be provided in positioning and stabilising structure 11300, for example in upper textile portion 11310. The EEG sensor may be partially exposed in the manner shown at module 11354 in Fig. 9C such that it can contact the skin of the patient's forehead. Typically, the EEG sensor comprises a plurality of EEG electrodes that generate signals that may be analysed to detect sleep stages. The signals may be transmitted (via transceiver 11350) to an external device, such as the patient's smartphone, and the sleep stage, cycle and duration information may be used to provide feedback to the patient on how well the sleep therapy is progressing, as well as recommendations for enhanced health. For example, the EEG sensor measurements may be used for accurate sleep staging, to enable a more accurate determination of when an apnea or arousal from sleep occurs, e.g. during a sleep study.

In some forms of the present technology, the sleep stage information may be transmitted to RPT device 4000, such that it can be used by pressure generator 4140 to adjust the therapy pressures to avoid arousal or obstruction events.

In some forms of the present technology, the sleep stage information may be used to activate sleep-enhancing white/pink noise, and/or binaural beats. These may be produced by audio devices embedded in the patient interface 11000 itself, or by external devices that receive trigger signals from the patient interface 11000 via transceiver 11350. For example, one or more miniature bone-conduction speakers may be incorporated at temple regions of the upper textile portion 11310.

In some forms of the present technology, a positioning and stabilising structure 11300 may incorporate electromyography (EMG) and/or electrooculography (EOG) sensors. EMG and EOG sensor signals may be analysed to determine REM sleep stage occurrences. In similar fashion to examples that incorporate EEG sensors, the sleep stage information determined by the EMG/EOG sensors may be used to provide feedback to the patient on how well sleep therapy is progressing, and may also be used to regulate pressure generator 4140 to avoid arousal or obstruction events, or to activate one or more audio devices to produce sleep-enhancing noise.

At least some of the EMG/EOG sensors may be incorporated in upper textile portion 11310. For example, a ground electrode and reference electrode may be provided in the upper textile portion 11310, for example in a front section thereof, and exposed through respective apertures in inner layer 11370 so as to be able to contact the patient's forehead. In another example, a ground electrode may be provided in a rear section 11306 of upper textile portion 11310, or in second lower textile portion 11308 such that the ground electrode sits behind the patient's ear in use. Further electrodes may be provided, each having a cable that attaches to and/or extends within upper textile portion 11310 or first lower textile portion 11320 or second lower textile portion 11308 at one end, and to an electrode patch at the other end, the electrode patch being positionable by the patient on the temple and below their eye to provide two additional measurement channels.

In some forms of the present technology, a microphone, such as a MEMS microphone or Electret microphone, may be incorporated in a patient interface 11000 to detect snoring. For example, the microphone may be located in or on an internal surface of plenum chamber 11200, or on an external surface thereof, adjacent the patient's nares. The microphone may be coupled to a communications interface to enable communication of data to the pressure generator 4140 of RPT device 4000, to regulate the pressure produced thereby. For example, when a light snoring noise pattern is detected, the therapy pressure can be gradually increased to prevent an obstructive event. When the snoring noise pattern subsides, the therapy pressure can be ramped down.

In some forms of the present technology, a patient interface 11000 may incorporate a humidity sensor and temperature sensor, for example on an internal surface of plenum chamber 11200, to monitor the temperature and humidity inside the plenum chamber 11200. The sensors may be coupled to a communications interface for transmitting humidity and temperature data to RPT device 4000 and humidifier 5000 to regulate the operation thereof. The pressure generator 4140 and humidifier 5000 power may be regulated to prevent condensate accumulation. For example, the humidifier 5000 may be activated in stages, and/or heater power levels may be controlled, followed by flushing with plain air, still keeping moisture levels (as measured by the humidity sensor) sufficient to prevent dry mouth.

In some forms of the present technology, a pressure sensor may be provided inside plenum chamber 11200, for example on an inner surface thereof. This enables the air pressure inside plenum chamber 11200 to be monitored and a signal sent to the pressure generator 4140 to adjust pressure and flow dynamically. This may optimise the pressure generator 4140 response to the patient's breathing pattern.

In some forms of the present technology, a $CO_2$ sensor may be provided inside plenum chamber 11200. For example, the $CO_2$ level inside the plenum chamber 11200 may be monitored. When a slight increase in $CO_2$ level is detected, an electromechanical vent (not shown in Fig. 9A-9C) may be opened to allow higher flushing of air from the plenum chamber 11200. Further, a signal may be sent to the pressure generator 4140 to slightly increase the flow to flush out $CO_2$ when the $CO_2$ level increases slightly. This may be done dynamically so as to minimise patient discomfort.

In some forms of the present technology, a combination of sensors and actuators may be provided to effect localised temperature change to improve patient comfort. For example, an EEG sensor and/or pulse oximeter may be provided in upper textile portion 11310 (for example in the manner shown at 11354 in Fig. 9C), and a temperature sensor and/or a humidity sensor may also be provided in upper textile portion 11310 (for example in the manner shown at 11358 in Fig. 9C). Signals from the EEG and/or PPG sensors may be analysed to detect sleep state, and signals from the temperature sensor and/or humidity sensor may be used to assess ambient comfort levels. One or more Peltier elements may be provided, for example in wearable form on a wristband, and may be coupled to circuitry that communicates with the EEG/PPG and temperature/humidity sensors to receive signals indicative of sleep state and ambient comfort level, and that causes the Peltier element to be activated to locally warm or cool the body (e.g. at the wrist) to help the patient remain in a comfortable sleep state.

In some forms of the present technology, a haptic feedback element (such as a miniature vibratory motor) may be incorporated in the patient interface 11000, for example in a temple region of the positioning and stabilising structure 11300 (e.g. of upper textile portion 11310). The haptic feedback element may deliver vibrations to the patient to produce a calming effect. For example, processor 11350 may monitor heart rate data from a pulse oximeter 11354, and if this exceeds a threshold, transmit a trigger signal to the haptic feedback element to cause it to vibrate at a few beats lower than the patient's current heart rate, to help slow it down. In another example, as mentioned above, a haptic feedback element may be used to influence the sleeping position of the patient if it is detected that they are in a sleeping position that is correlated with apnea or hypopnea events.

In some forms of the present technology, one or more miniature thermo electric generators (TEGs) may be incorporated into the patient interface 11000, such that the difference between the patient's body temperature and the ambient temperature may be used to generate a potential difference and thus to provide power to the various electronic components (sensors, actuators, processor, etc.) of the patient interface 11000. For example, miniature TEGs may be located in the upper textile portion 11310, and exposed through an aperture of the inner layer 11370 such that they contact the patient's forehead.

In some forms of the present technology, multiple sensors may be incorporated in a single module. For example, an accelerometer and gyroscope may be incorporated in a single package.

While the various sensors and actuators have been described as being incorporated in the patient interface 11000 shown in Fig. 9A-9C, it will be appreciated that they may be incorporated in like fashion in any of the other patient interfaces 3000, 6000, 7000, 8000, 9000, 10000 disclosed herein.

### 5.3.4 Vent

In one form, the patient interface 3000 includes a vent 3400, 10400 constructed and arranged to allow for the washout of exhaled gases, e.g. carbon dioxide.

In certain forms the vent 3400, 10400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled $CO_2$ by the patient while maintaining the therapeutic pressure in the plenum chamber in use.

One form of vent 3400, 10400 in accordance with the present technology comprises a plurality of holes, for example, about 20 to about 80 holes, or about 40 to about 60 holes, or about 45 to about 55 holes.

The vent 3400, 10400 may be located in the plenum chamber 3200, 10200. Alternatively, the vent 3400, 10400 is located in a decoupling structure, e.g., a swivel.

In some forms, the vent 10400 may be a transparent mesh with in-built venting.

Although not explicitly shown in Figs. 4B-4D, 5A-5B, 6, 7A-7C and 8B, it will be appreciated that the plenum chamber of each of the examples shown in those figures will also typically include a vent to enable flushing of $CO_2$ and other exhaled gases.

In some forms of the present technology, the vent 3400, 10400 may be an active vent, and may be activated in accordance with sensor measurements from one or more sensors of the patient interface (e.g. patient interface 10000). For example, in some forms the plenum chamber 10200 may comprise one or more of a $CO_2$ sensor, a temperature sensor, and a humidity sensor, and detection of one or more of these quantities above respective thresholds may trigger opening of the vent.

### 5.3.5 Decoupling structure(s)

In one form the patient interface 3000 includes at least one decoupling structure, for example, a swivel or a ball and socket.

### 5.3.6 Connection port

Connection port 3600, 6600, 7600, 8600, 9600, 10600, 11600 allows for connection to the air circuit 4170. In some examples, a connection port 11600 may be integrated with a positioning and stabilising structure 11300, as shown in Fig. 9A for example.

A number of different forms of connection between air circuit 4170 and connection port 3600, 6600, 7600, 8600, 9600, 10600, 11600 are possible, provided that a substantially air-tight seal is formed to substantially prevent pressure leakage during therapy. As mentioned in relation to the port 11600, magnetic elements may be located at an interior surface of the connection port for connection to corresponding magnetic elements located on a connector at one end of air circuit 4170. In another example, the connector of the air circuit may attach to the connection port by a snap-fit (such as an annular snap-fit or cantilever snap-fit, either of which may be rigid-to-rigid or rigid-to-resilient) or friction fit.

### 5.3.7 Forehead support

In one form, the patient interface 3000 includes a forehead support 3700.

### 5.3.8 Anti-asphyxia valve

In one form, the patient interface 3000 includes an anti-asphyxia valve.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

## 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH$_2$O, or at least 10cmH$_2$O, or at least 20 cmH$_2$O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH$_2$O to about 20 cmH$_2$O, or in other forms up to about 30 cmH$_2$O when delivering respiratory pressure therapy. The blower may be as described in any one of the following patents or patent applications the contents of which are incorporated herein by reference in their entirety: U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

#### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 and representing a pressure is received by the central controller 4230.

#### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

#### 5.4.2.1 Power supply

A power supply 4210 may be located internal or external of the external housing 4010 of the RPT device 4000.
In one form of the present technology, power supply 4210 provides electrical power to the RPT device 4000 only. In another form of the present technology, power supply 4210 provides electrical power to both RPT device 4000 and humidifier 5000.

#### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.
In one form, the input device 4220 may be constructed and arranged to allow a person to select a value and/or a menu option.

#### 5.4.2.3 Central controller

In one form of the present technology, the central controller 4230 is one or a plurality of processors suitable to control an RPT device 4000.
Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.
In one form of the present technology, the central controller 4230 is a dedicated electronic circuit.
In one form, the central controller 4230 is an application-specific integrated circuit. In another form, the central controller 4230 comprises discrete electronic components.
The central controller 4230 may be configured to receive input signal(s) from one or more transducers 4270, one or more input devices 4220, and the humidifier 5000.
The central controller 4230 may be configured to provide output signal(s) to one or more of an output device 4290, a therapy device controller 4240, a data communication interface 4280, and the humidifier 5000.
In some forms of the present technology, the central controller 4230 is configured to implement the one or more methodologies described herein, such as the one or more algorithms 4300 which may be implemented with processor-control instructions, expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

#### 5.4.2.4 Clock

The RPT device 4000 may include a clock 4232 that is connected to the central controller 4230.

#### 5.4.2.5 Therapy device controller

In one form of the present technology, therapy device controller 4240 is a therapy control module 4330 that forms part of the algorithms 4300 executed by the central controller 4230.
In one form of the present technology, therapy device controller 4240 is a dedicated motor control integrated circuit. For example, in one form a MC33035 brushless DC motor controller, manufactured by ONSEMI is used.

#### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally, or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### 5.4.2.9.1 Display driver

A display driver 4292 receives as an input the characters, symbols, or images intended for display on the display 4294, and converts them to commands that cause the display 4294 to display those characters, symbols, or images.

#### 5.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure $Pm$, the vent flow rate $Qv$, the respiratory flow rate $Qr$, and the leak flow rate $Ql$.

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

### 5.4.3.1.1 Interface pressure estimation

In one form of the present technology, an interface pressure estimation algorithm 4312 receives as inputs a signal from the pressure sensor 4272 indicative of the pressure in the pneumatic path proximal to an outlet of the pneumatic block (the device pressure Pd) and a signal from the flow rate sensor 4274 representative of the flow rate of the airflow leaving the RPT device 4000 (the device flow rate $Qd$). The device flow rate $Qd$, absent any supplementary gas 4180, may be used as the total flow rate $Qt$. The interface pressure algorithm 4312 estimates the pressure drop $\Delta P$ through the air circuit 4170. The dependence of the pressure drop $\Delta P$ on the total flow rate $Qt$ may be modelled for the particular air circuit 4170 by a pressure drop characteristic $\Delta P(Q)$. The interface pressure estimation algorithm, 4312 then provides as an output an estimated pressure, $Pm$, in the patient interface 3000. The pressure, $Pm$, in the patient interface 3000 may be estimated as the device pressure $Pd$ minus the air circuit pressure drop $\Delta P$.

### 5.4.3.1.2 Vent flow rate estimation

In one form of the present technology, a vent flow rate estimation algorithm 4314 receives as an input an estimated pressure, $Pm$, in the patient interface 3000 from the interface pressure estimation algorithm 4312 and estimates a vent flow rate of air, $Qv$, from a vent 3400 in a patient interface 3000. The dependence of the vent flow rate $Qv$ on the interface pressure $Pm$ for the particular vent 3400 in use may be modelled by a vent characteristic $Qv(Pm)$.

### 5.4.3.1.3 Leak flow rate estimation

In one form of the present technology, a leak flow rate estimation algorithm 4316 receives as an input a total flow rate, $Qt$, and a vent flow rate $Qv$, and provides as an output an estimate of the leak flow rate $Ql$. In one form, the leak flow rate estimation algorithm estimates the leak flow rate $Ql$ by calculating an average of the difference between total flow rate $Qt$ and vent flow rate $Qv$ over a period sufficiently long to include several breathing cycles, e.g. about 10 seconds.

In one form, the leak flow rate estimation algorithm 4316 receives as an input a total flow rate $Qt$, a vent flow rate $Qv$, and an estimated pressure, $Pm$, in the patient interface 3000, and provides as an output a leak flow rate $Ql$, by calculating a leak conductance, and determining a leak flow rate $Ql$ to be a function of leak conductance and pressure, $Pm$. Leak conductance is calculated as the quotient of low pass filtered non-vent flow rate equal to the difference between total flow rate $Qt$ and vent flow rate $Qv$, and low pass filtered square root of pressure $Pm$, where the low pass filter time constant has a value sufficiently long to include several breathing cycles, e.g. about 10 seconds. The leak flow rate $Ql$ may be estimated as the product of leak conductance and a function of pressure, $Pm$.

### 5.4.3.1.4 Respiratory flow rate estimation

In one form of the present technology, a respiratory flow rate estimation algorithm 4318 receives as an input a total flow rate, $Qt$, a vent flow rate, $Qv$, and a leak flow rate, $Ql$, and estimates a respiratory flow rate of *air, Qr,* to the patient, by subtracting the vent flow rate $Qv$ and the leak flow rate $Ql$ from the total flow rate $Qt$.

**5.4.3.2 Therapy Engine Module**

In one form of the present technology, a therapy engine module 4320 receives as inputs one or more of a pressure, *Pm,* in a patient interface 3000, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

*5.4.3.2.1 Phase determination*

In one form of the present technology, the RPT device 4000 does not determine phase.

In one form of the present technology, a phase determination algorithm 4321 receives as an input a signal indicative of respiratory flow rate, *Qr,* and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate *Qr* has a value that exceeds a positive threshold, and a discrete value of 0.5 revolutions (thereby "cycling" the RPT device 4000) when a respiratory flow rate *Qr* has a value that is more negative than a negative threshold. The inhalation time *Ti* and the exhalation time *Te* may be estimated as typical values over many respiratory cycles of the time spent with phase $\Phi$ equal to 0 (indicating inspiration) and 0.5 (indicating expiration) respectively.

Another implementation of discrete phase determination provides a trivalued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate *Qr.* A continuous value of phase determined in this implementation is often referred to as "fuzzy phase". In one implementation of a fuzzy phase determination algorithm 4321, the following rules are applied to the respiratory flow rate *Qr:*

    1. If the respiratory flow rate is zero and increasing fast then the phase is 0 revolutions.

    2. If the respiratory flow rate is large positive and steady then the phase is 0.25 revolutions.

    3. If the respiratory flow rate is zero and falling fast, then the phase is 0.5 revolutions.

    4. If the respiratory flow rate is large negative and steady then the phase is 0.75 revolutions.

    5. If the respiratory flow rate is zero and steady and the 5-second low-pass filtered absolute value of the respiratory flow rate is large then the phase is 0.9 revolutions.

    6. If the respiratory flow rate is positive and the phase is expiratory, then the phase is 0 revolutions.

    7. If the respiratory flow rate is negative and the phase is inspiratory, then the phase is 0.5 revolutions.

    8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate $Qr$ as described above, as are the inhalation time $Ti$ and the exhalation time $Te$. The continuous phase $\Phi$ at any instant may be determined as the half the proportion of the inhalation time $Ti$ that has elapsed since the previous trigger instant, or 0.5 revolutions plus half the proportion of the exhalation time $Te$ that has elapsed since the previous cycle instant (whichever instant was more recent).

### 5.4.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure $Pt$ that varies as a function of phase $\Phi$ of a respiratory cycle of a patient according to a waveform template $\Pi(\Phi)$.

In one form of the present technology, a waveform determination algorithm 4322 provides a waveform template $\Pi$ ($\Phi$) with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi$ ($\Phi$) in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi = 0$ revolutions) or exhalation ($\Phi = 0.5$ revolutions), the waveform determination algorithm 4322 computes a waveform template $\Pi$ "on the fly" as a function of both discrete phase $\Phi$ and time t measured since the most recent trigger instant. In one such form, the waveform determination algorithm 4322 computes the waveform template $\Pi(\Phi, t)$ in two portions (inspiratory and expiratory) as follows:

$$\Pi\left(\Phi, t\right) = \begin{cases} \Pi_i\left(t\right), & \Phi = 0 \\ \Pi_e\left(t - T_i\right), & \Phi = 0.5 \end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 5.4.3.2.3 Ventilation determination

In one form of the present technology, a ventilation determination algorithm 4323 receives an input a respiratory flow rate $Qr$, and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, $Qr$, optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle *K* proportion (by time) of the inspiratory portion, where $0 < K < 1$. There is an arbitrarily large number of measures that are exactly proportional to ventilation if the flow rate shape is constant.

### 5.4.3.2.4 Determination of Inspiratory Flow limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal *Qr* and provides as an output a metric of the extent to which the inspiratory portion of the breath exhibits inspiratory flow limitation.

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as *Qs(t).* Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flowlimited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 5.4.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 5.4.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal $Qr$ and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal $Qr$ to reduce background noise, e.g., the sound of airflow in the system from the blower.

### 5.4.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal $Qr$, and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure $Pt.$ In one implementation, the forced oscillation is of frequency 2 Hz with amplitude about 1 $cmH_2O$.

In one form, airway patency determination algorithm 4327 receives as an input a respiratory flow rate signal $Qr$, and determines the presence or absence of a cardiogenic signal. The absence of a cardiogenic signal is taken to be an indication of a closed airway.

### 5.4.3.2.8 Determination of target ventilation

In one form of the present technology, the central controller 4230 takes as input the measure of current ventilation, $Vent$, and executes one or more target ventilation determination algorithms 4328 for the determination of a target value $Vtgt$ for the measure of ventilation.

In some forms of the present technology, there is no target ventilation determination algorithm 4328, and the target value $Vtgt$ is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value $Vtgt$ from a value $Vtyp$ indicative of the typical recent ventilation of the patient.

In some forms of adaptive servo-ventilation, the target ventilation $Vtgt$ is computed as a high proportion of, but less than, the typical recent ventilation $Vtyp$. The high proportion in such forms may be in the range (80%, 100%), or (85%, 95%), or (87%, 92%).

In other forms of adaptive servo-ventilation, the target ventilation $Vtgt$ is computed as a slightly greater than unity multiple of the typical recent ventilation $Vtyp$.

The typical recent ventilation $Vtyp$ is the value around which the distribution of the measure of current ventilation $Vent$ over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation $Vtyp$ from the measure of current ventilation, $Vent$. One such measure is the output of a low-pass filter on the measure of current ventilation $Vent$, with time constant equal to one hundred seconds.

### 5.4.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure $Pt.$ In one implementation of this form, the therapy parameter determination algorithm 4329 determines the treatment pressure *Pt* using the equation

$$Pt = A\Pi(\Phi, t) + P_0 \qquad\qquad (1)$$

where:

- A is the amplitude,
- $\Pi(\Phi, t)$ is the waveform template value (in the range 0 to 1) at the current value $\Phi$ of phase and t of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude *A* and the base pressure $P_0$ may be set by the therapy parameter determination algorithm 4329 depending on the chosen respiratory pressure therapy mode in the manner described below.

### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3000 is equal to the treatment pressure *Pt.*

### 5.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

**5.5.1 Supplementary gas delivery**

In one form of the present technology, supplementary gas, e.g. oxygen, 4180 is delivered to one or more points in the pneumatic path, such as upstream of the pneumatic block 4020, to the air circuit 4170, and/or to the patient interface 3000.

5.6 HUMIDIFIER

**5.6.1 Humidifier overview**

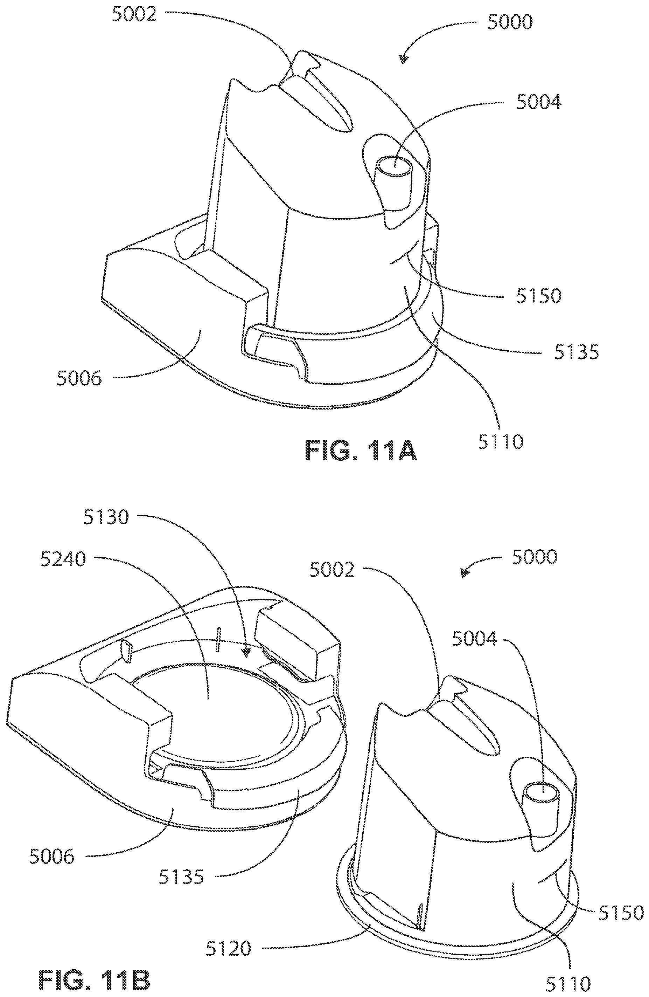

In one form of the present technology there is provided a humidifier 5000 (e.g. as shown in Fig. 11A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 11A and Fig. 11B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

**5.6.2 Humidifier components**

**5.6.2.1 Water reservoir**

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 11A and Fig. 11B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

**5.6.2.2 Conductive portion**

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

**5.6.2.3 Humidifier reservoir dock**

In one form, the humidifier 5000 may comprise a humidifier reservoir dock 5130 (as shown in Fig. 11B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 11A-11B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.2.5 Humidifier transducer(s)

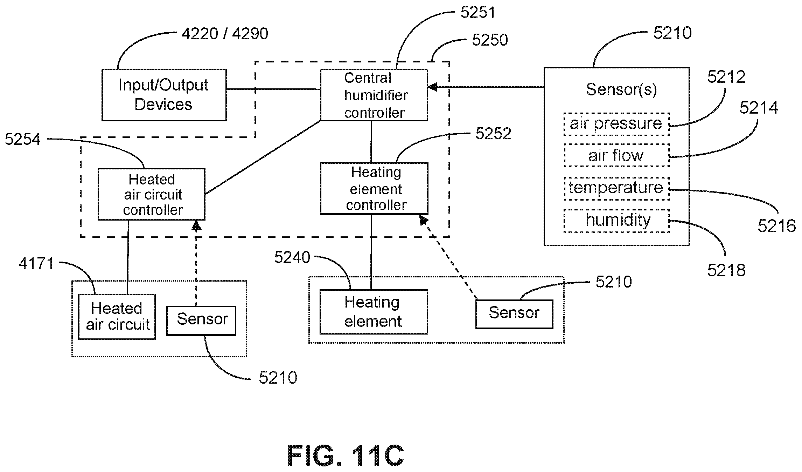

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 11C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

#### 5.6.2.5.1 Pressure transducer

One or more pressure transducers 5212 may be provided to the humidifier 5000 in addition to, or instead of, a pressure sensor 4272 provided in the RPT device 4000.

#### 5.6.2.5.2 Flow rate transducer

One or more flow rate transducers 5214 may be provided to the humidifier 5000 in addition to, or instead of, a flow rate sensor 4274 provided in the RPT device 4000.

#### 5.6.2.5.3 Temperature transducer

The humidifier 5000 may comprise one or more temperature transducers 5216. The one or more temperature transducers 5216 may be configured to measure one or more temperatures such as of the heating element 5240 and/or of the flow of air downstream of the humidifier outlet 5004. In some forms, the humidifier 5000 may further comprise a temperature sensor 5216 to detect the temperature of the ambient air.

#### 5.6.2.5.4 Humidity transducer

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

### 5.6.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072, which is incorporated herewith by reference in its entirety.

In some forms, the heating element 5240 may be provided in the humidifier base 5006 where heat may be provided to the humidifier reservoir 5110 primarily by conduction as shown in Fig. 11B.

### 5.6.2.7 Humidifier controller

According to one arrangement of the present technology, a humidifier 5000 may comprise a humidifier controller 5250 as shown in Fig. 11C. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 11C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

5.7 SCREENING, DIAGNOSIS, MONITORING SYSTEMS

**5.7.1 Overview**

At least some forms of the present technology allow for screening, diagnosis and/or monitoring of sleep health using sensors incorporated in a patient interface. The sensors may be provided in a positioning and stabilising structure of the patient interface, such as the positioning and stabilising structure 11300 of Fig. 9A and 9B, and/or in a plenum chamber of the patient interface.

For example, as mentioned, a sensor-enabled positioning and stabilising structure in the form of a headband, such as positioning and stabilising structures 6300 or 11300, may be worn as a headband by the patient prior to commencing therapy, and sensor measurements recorded during sleep. The sensor measurements may be used to accurately monitor sleep stages and sleeping positions, to track vital signs and other physiological indicators, and to detect apnea and/or hypopnea events. The sleep data and physiological data may be used by clinicians to diagnose sleep disorders and to recommend appropriate therapy. Further, the same parameters may be monitored by the sensors during therapy, and compared to the parameters prior to commencing therapy (or at an earlier stage of therapy) to enable the patient and the clinician to assess the efficacy of the therapy.

As mentioned, physiological and sleep data may be recorded by one or more sensors of the patient interface, and communicated to external computing devices such as a smartphone of the patient, and/or a monitoring server that is operated by or accessible to a clinician or other healthcare provider.

In some forms of the present technology, a pulse oximeter incorporated in the positioning and stabilising structure 11300 may be used to determine blood oxygen saturation level and heart rate during the period that the patient interface 11000 is worn, and this data may be transmitted to an external computing device such as a smartphone, other mobile computing device, or laptop or desktop computing system of the patient. The data may comprise time series data that may be consolidated to provide feedback to the patient on their health levels, and recommendations for follow-up (for example, by a clinician). This may, for example, be based on determination of an AHI as discussed above, which can be used in conjunction with other sensor measurements to determine when and how often apnea events are occurring. The data may further be used to devise an optimal treatment regimen for the patient.

Other sensors which may be incorporated in a screening, diagnosis and/or monitoring system that comprises a sensor-enabled patient interface include, without limitation: an EEG sensor for detection of sleep stages; EMG and EOG sensors for determining REM sleep stage occurrences; a microphone for detecting snoring or other sounds indicative of disturbed sleep; a humidity sensor, temperature sensor, pressure sensor, and/or $CO_2$ sensor, each provided internally of a plenum chamber 11200 of the patient interface.

Some example applications of sensor-enabled patient interfaces will now be described.

**5.7.2 Polysomnography**

Polysomnography (PSG) is a monitoring process that typically involves a variety of different sensors and associated equipment, and that can be challenging to set up, even for an expert. A typical PSG system comprises a headbox which receives and records signals from the following sensors: an EOG electrode; an EEG electrode; an ECG electrode; a submental EMG electrode; a snore sensor; a respiratory inductance plethysmogram (respiratory effort sensor) on a chest band; a respiratory inductance plethysmogram (respiratory effort sensor) on an abdominal band; an oro-nasal cannula with oral thermistor; a photoplethysmograph (pulse oximeter); and a body position sensor. The electrical signals are referred to a ground electrode (ISOG) positioned in the centre of the forehead.

A sensor-enabled patient interface, such as patient interface 11000, can replace some or all of the functions of an existing PSG system, since, as discussed above, some or all of the sensors used by a PSG system can be provided in the positioning and stabilising structure (e.g. 11300) and/or in the plenum chamber (e.g. 11200). For example, EOG, EEG, ECG and EMG electrodes, a microphone (acting as a snore sensor), a PPG sensor, and an accelerometer and/or gyroscope (acting as body position sensor(s)) can all be provided in upper textile portion 11310 and/or lower textile portions 11308, 11320 of the positioning and stabilising structure 11300. A ground electrode may also be provided (e.g. in the lower textile portion 11308 for placement behind the patient's ear, as described above).

By integrating the sensors with the patient interface 11000, in at least some examples, it becomes far more straightforward to implement PSG, since the patient is simply able to wear the patient interface 11000 in the manner in which they would do so for therapy, with no or minimal additional configuration required (e.g., no need to manually place various electrodes for the EEG/EMG/EOG sensors).

### 5.7.3 Non-obtrusive monitoring system

In one example, one or more accelerometers and/or one or more gyroscopes and/or one or more other motion sensors may be provided in the positioning and stabilising structure 11300. The motion sensor(s) is or are configured to generate one or more signals representing bodily movement of the patient, from which may be obtained a signal representing respiratory movement of the patient.

### 5.7.4 Respiratory polygraphy

Respiratory polygraphy (RPG) is a term for a simplified form of PSG without the electrical signals (EOG, EEG, EMG), snore, or body position sensors. Ordinarily, RPG comprises at least a thoracic movement signal from a respiratory inductance plethysmogram (movement sensor) on a chest band, a nasal pressure signal sensed via a nasal cannula, and an oxygen saturation signal from a pulse oximeter, e.g. the pulse oximeter. The three RPG signals, or channels, are received by an RPG headbox.

A sensor-enabled patient interface, such as patient interface 11000, can replace some or all of the functions of an existing RPG system. For example, accelerometer and/or gyroscope measurements from sensors mounted in headgear 11300 may be used as a proxy for a thoracic movement signal. A nasal pressure signal may be measured by a pressure sensor mounted inside the plenum chamber, for example on the internal surface thereof so as to lie adjacent to the patient's nares in use. An oxygen saturation signal may be measured by a PPG sensor mounted in headgear 11300, for example as shown at 11355 in Fig. 9C. The proxy thoracic movement signal, the nasal pressure signal, and the oxygen saturation signal may be received by an on-board processor 11350 of the patient interface 11000, and/or may be transmitted to an external computing device for analysis in the same manner as conventional RPG signals.

In certain configurations, a nasal pressure signal is a satisfactory proxy for a nasal flow rate signal generated by a flow rate transducer in-line with a sealed nasal mask, in that the nasal pressure signal is comparable in shape to the nasal flow rate signal. The nasal flow rate in turn is equal to the respiratory flow rate if the patient's mouth is kept closed, i.e. in the absence of mouth leaks.

### 5.8 PORTABLE OXYGEN CONCENTRATOR (POC)

Portable oxygen concentrators may take advantage of pressure swing adsorption (PSA). Pressure swing adsorption may involve using one or more compressors to increase gas pressure inside a canister that contains particles of a gas separation adsorbent arranged in a "sieve bed". As the pressure increases, certain molecules in the gas may become adsorbed onto the gas separation adsorbent. Removal of a portion of the gas in the canister under the pressurized conditions allows separation of the non-adsorbed molecules from the adsorbed molecules. The gas separation adsorbent may be regenerated by reducing the pressure, which reverses the adsorption of molecules from the adsorbent. Further details regarding oxygen concentrators may be found, for example, in U.S. Published Patent Application No. 2009-0065007, published March 12, 2009, and entitled "Oxygen Concentrator Apparatus and Method", which is incorporated herein by reference.

Ambient air usually includes approximately 78% nitrogen and 21% oxygen with the balance comprised of argon, carbon dioxide, water vapor and other trace gases. If a gas mixture such as air, for example, is passed under pressure through a canister containing a gas separation adsorbent bed that attracts nitrogen more strongly than it does oxygen, part or all of the nitrogen will stay in the bed, and the gas coming out of the canister will be enriched in oxygen. When the bed reaches the end of its capacity to adsorb nitrogen, it can be regenerated by reducing the pressure, thereby releasing the adsorbed nitrogen. It is then ready for another cycle of producing oxygen enriched air. By alternating canisters in a two-canister system, one canister can be separating oxygen while the other canister is being purged (resulting in a continuous separation of the oxygen from the nitrogen). In this manner, oxygen enriched air can be accumulated, such as in a storage container or other pressurizable vessel or conduit coupled to the canisters, for a variety of uses including providing supplemental oxygen to patients.

### 5.9 RESPIRATORY THERAPY MODES

Various respiratory therapy modes may be implemented by the disclosed respiratory therapy system.

### 5.9.1 CPAP therapy

In some implementations of respiratory pressure therapy, the central controller 4230 sets the treatment pressure $Pt$ according to the treatment pressure equation (1) as part of the therapy parameter determination algorithm 4329. In one such implementation, the amplitude A is identically zero, so the treatment pressure $Pt$ (which represents a target value to be achieved by the interface pressure $Pm$ at the current instant of time) is identically equal to the base pressure $P_0$ throughout the respiratory cycle. Such implementations are generally grouped under the heading of CPAP therapy. In such implementations, there is no need for the therapy engine module 4320 to determine phase $\Phi$ or the waveform template $\Pi$ ($\Phi$).

In CPAP therapy, the base pressure $P_0$ may be a constant value that is hard-coded or manually entered to the RPT device 4000. Alternatively, the central controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

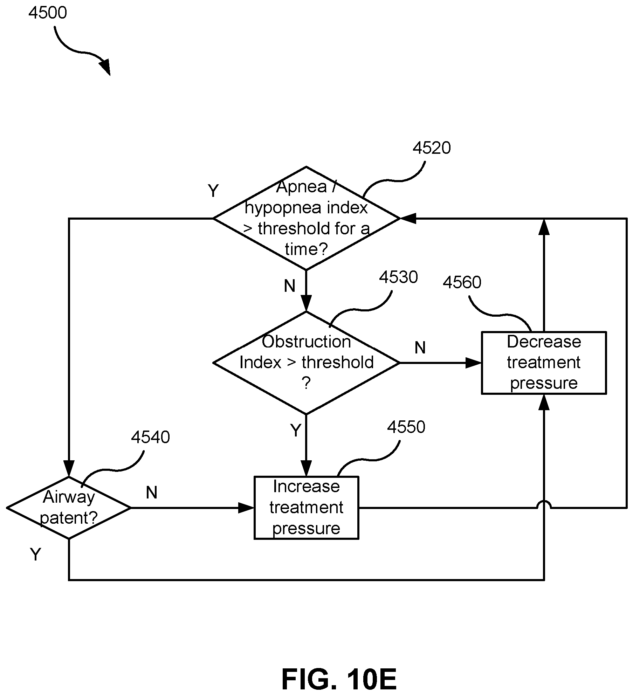

Fig. 10E is a flow chart illustrating a method 4500 carried out by the central controller 4230 to continuously compute the base pressure $P_0$ as part of an APAP therapy implementation of the therapy parameter determination algorithm 4329, when the pressure support A is identically zero.

The method 4500 starts at step 4520, at which the central controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure $P_0$ by a predetermined pressure increment $\Delta P$, provided the resulting treatment pressure $Pt$ would not exceed a maximum treatment pressure $Pmax$. In one implementation, the predetermined pressure increment $\Delta P$ and maximum treatment pressure $Pmax$ are 1 cmH$_2$O and 25 cmH$_2$O respectively. In other implementations, the pressure increment $\Delta P$ can be as low as 0.1 cmH$_2$O and as high as 3 cmH$_2$O, or as low as 0.5 cmH$_2$O and as high as 2 cmH$_2$O. In other implementations, the maximum treatment pressure $Pmax$ can be as low as 15 cmH$_2$O and as high as 35 cmH$_2$O, or as low as 20 cmH$_2$O and as high as 30 cmH$_2$O. The method 4500 then returns to step 4520.

At step 4560, the central controller 4230 decreases the base pressure $P_0$ by a decrement, provided the decreased base pressure $P_0$ would not fall below a minimum treatment pressure $Pmin$. The method 4500 then returns to step 4520. In one implementation, the decrement is proportional to the value of $P_0$-$Pmin$, so that the decrease in $P_0$ to the minimum treatment pressure $Pmin$ in the absence of any detected events is exponential. In one implementation, the constant of proportionality is set such that the time constant $\tau$ of the exponential decrease of $P_0$ is 60 minutes, and the minimum treatment pressure $Pmin$ is 4 cmH$_2$O. In other implementations, the time constant $\tau$ could be as low as 1 minute and as high as 300 minutes, or as low as 5 minutes and as high as 180 minutes. In other implementations, the minimum treatment pressure $Pmin$ can be as low as 0 cmH$_2$O and as high as 8 cmH$_2$O, or as low as 2 cmH$_2$O and as high as 6 cmH$_2$O. Alternatively, the decrement in $P_0$ could be predetermined, so the decrease in $P_0$ to the minimum treatment pressure $Pmin$ in the absence of any detected events is linear.

### 5.9.2 Bi-level therapy

In other implementations of this form of the present technology, the value of amplitude A in equation (1) may be positive. Such implementations are known as bi-level therapy, because in determining the treatment pressure $Pt$ using equation (1) with positive amplitude A, the therapy parameter determination algorithm 4329 oscillates the treatment pressure $Pt$ between two values or levels in synchrony with the spontaneous respiratory effort of the patient 1000. That is, based on the typical waveform templates $\Pi(\Phi, t)$ described above, the therapy parameter determination algorithm 4329 increases the treatment pressure $Pt$ to $P_0$ + A (known as the IPAP) at the start of, or during, or inspiration and decreases the treatment pressure $Pt$ to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude A, which has a "small" value (a few cmH$_2$O) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude A is large enough that the RPT device 4000 does some or all of the work of breathing of the patient 1000. In such forms, known as pressure support ventilation therapy, the amplitude A is referred to as the pressure support, or swing. In pressure support ventilation therapy, the IPAP is the base pressure $P_0$ plus the pressure support A, and the EPAP is the base pressure $P_0$.

In some forms of pressure support ventilation therapy, known as fixed pressure support ventilation therapy, the pressure support A is fixed at a predetermined value, e.g. 10 cmH$_2$O. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure support A so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support A is repeatedly computed as:

$$A = G \int (Vent - Vtgt)\, dt \qquad\qquad (2)$$

where *G* is the gain of the PI control. Larger values of gain *G* can result in positive feedback in the therapy engine module 4320. Smaller values of gain *G* may permit some residual untreated CSR or central sleep apnea. In some implementations, the gain *G* is fixed at a predetermined value, such as -0.4 cmH$_2$O/(L/min)/sec. Alternatively, the gain *G* may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations. In yet other implementations, the gain *G* may vary depending on the difference between the current measure *Vent* of ventilation and the target ventilation *Vtgt.*

Other servo-ventilation control methodologies that may be applied by the therapy parameter determination algorithm 4329 include proportional (P), proportional-differential (PD), and proportional-integral-differential (PID).

The value of the pressure support A computed via equation (**Error! Reference source not found**.2) may be clipped to a range defined as [*Amin, Amax*]. In this implementation, the pressure support A sits by default at the minimum pressure support *Amin* until the measure of current ventilation *Vent* falls below the target ventilation *Vtgt,* at which point A starts increasing, only falling back to *Amin* when *Vent* exceeds *Vtgt* once again.

The pressure support limits *Amin* and *Amax* are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

5.10 GLOSSARY

For the purposes of the present technology disclosure, in certain forms of the present technology, one or more of the following definitions may apply. In other forms of the present technology, alternative definitions may apply.

**5.10.1 General**

*Air:* In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol $Q$. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.

*Patient:* A person, whether or not they are suffering from a respiratory condition.

*Pressure:* Force per unit area. Pressure may be expressed in a range of units, including $cmH_2O$, g-f/cm$^2$ and hectopascal. 1 $cmH_2O$ is equal to 1 g-f/cm$^2$ and is approximately 0.98 hectopascal (1 hectopascal = 100 Pa = 100 N/m$^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 5.10.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240 - 15e1.

*Polycarbonate:* a thermoplastic polymer of Bisphenol-A Carbonate.

*Textile:* A flexible material formed from a network of fibres, which may be natural, artificial, or a combination thereof. The fibres (e.g. wool, flax, cotton, hemp, and/or artificial fibres) may be spun into a yarn that is woven, knitted, crocheted, knotted, tatted, felted, and/or braided to form the textile. As used herein, the terms "textile" and "fabric" are interchangeable.

### 5.10.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

**5.10.2 Respiratory cycle**

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

(i) *Flattened:* Having a rise followed by a relatively flat portion, followed by a fall.

(ii) *M-shaped:* Having two local peaks, one at the leading edge, and one at the trailing edge, and a relatively flat portion between the two peaks.

(iii) *Chair-shaped:* Having a single local peak, the peak being at the leading edge, followed by a relatively flat portion.

(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.10.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP):* Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., $PS = IPAP - EPAP$). In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 5.10.4 Anatomy

### 5.10.4.1 Anatomy of the face

*Ala:* the external outer wall or "wing" of each nostril (plural: alar)

*Alar angle:*

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Lip, lower (labrale inferius):*

*Lip, upper (labrale superius):*

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

**5.10.4.2 Anatomy of the skull**

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.10.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.10.5 Patient interface

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell*: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent*: (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

## 5.10.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

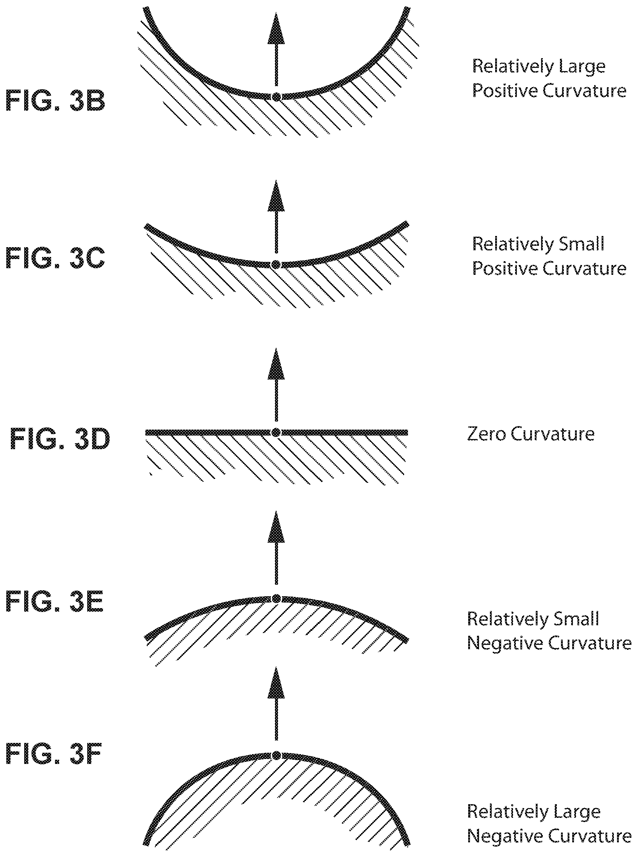

To facilitate describing the shape of the three-dimensional structures and the surfaces, we first consider a cross-section through a surface of the structure at a point, *p*. See Fig. 3B to Fig. 3F, which illustrate examples of cross-sections at point *p* on a surface, and the resulting plane curves. Figs. 3B to 3F also illustrate an outward normal vector at *p*. The outward normal vector at *p* points away from the surface. In some examples we describe the surface from the point of view of an imaginary small person standing upright on the surface.

## 5.10.6.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

## 5.10.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from $f(0)$ to $f(1)$ on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from $f(0)$ to $f(1)$, that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.10.6.3 Space curves

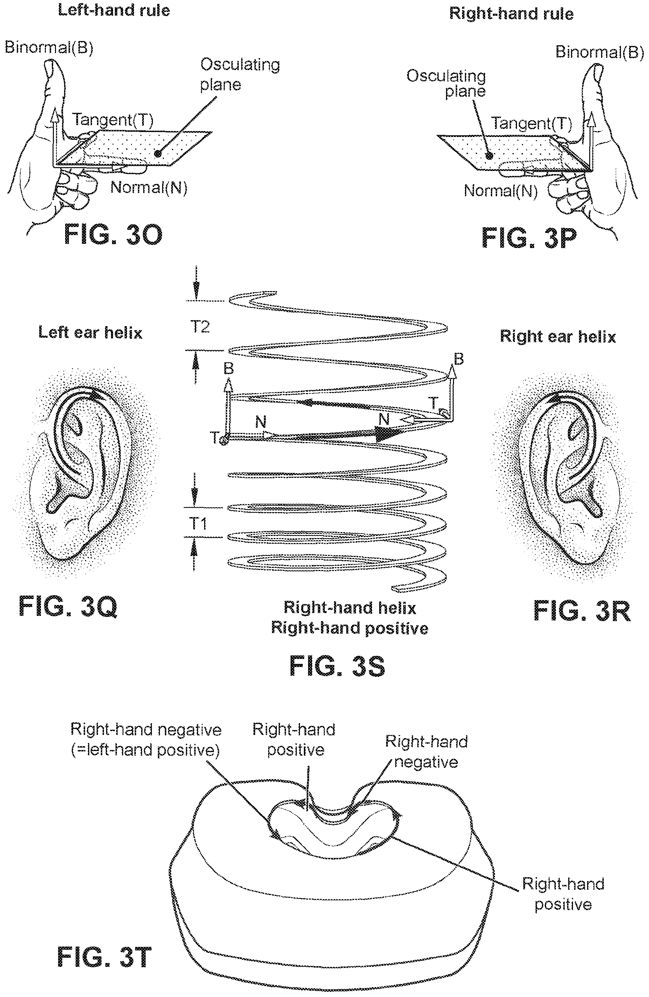

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since $T2>T1$, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.10.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

5.11 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

The following aspects are preferred embodiments of the invention.

1. A positioning and stabilising structure for a patient interface, comprising:

a front section and a rear section forming a continuous loop of material, the front section forming a first bifurcated section with a first part and a second part;
an upper textile portion comprising a resilient circumferential band for fitting to a patient's head in use, the upper textile portion formed from the rear section and from the first part ; and
at least one lower textile portion formed from the second part and being movably connected to the upper textile portion;

wherein at least a first lower textile portion of the at least one lower textile portion is stretchable relative to the upper textile portion, and is constructed and arranged to provide a force to hold a seal-forming structure of the patient interface in a therapeutically effective position on the patient's head; and

wherein the first lower textile portion is movable between a first position and a second position, the first lower textile portion configured to be proximate to the first part and to overlay the patient's frontal bone in the first position, and the first lower textile portion configured to be distal to the first part and overlay the patient's cheeks in the second position.

2. A positioning and stabilising structure according to aspect 1, wherein the first lower textile portion is integral with the upper textile portion.

3. A positioning and stabilising structure according to aspect 1 or aspect 2, wherein the positioning and stabilising structure is in the form of a headband.

4. A positioning and stabilising structure according to any one of aspects 1 to 3, wherein the rear section of the headband comprises a second bifurcated section comprising a second part of the upper textile portion and further comprising a second lower textile portion.

5. A positioning and stabilising structure according to aspect 4, wherein the second part of the upper textile portion and the second lower textile portion are configured to overlay the patient's occipital bone in the first position, and wherein the second part of the upper textile portion is movable away from the second lower textile portion to overlay the parietal bone in the second position.

6. A positioning and stabilising structure according to aspect 4 or aspect 5, wherein the front section and the rear section combine to form an X-shape in the second position.

7. A positioning and stabilising structure according to any one of aspects 1 to 6, wherein the first lower textile portion is a seal retention band that is resiliently stretchable along at least part of its length, and is adapted to engage with an outer surface of the patient interface to hold the seal-forming structure in the therapeutically effective position.

8. A positioning and stabilising structure according to aspect 7, wherein the seal retention band is more stretchable than the upper textile portion.

9. A positioning and stabilising structure according to aspect 7 or aspect 8, wherein the seal retention band is adapted to be received in a channel of the patient interface.

10. A positioning and stabilising structure according to aspect 9, wherein the channel is formed in a plenum chamber of the patient interface.

11. A positioning and stabilising structure according to any one of aspects, 7 to 10, wherein the seal retention band comprises a port for coupling the seal-forming structure to an air circuit for supplying pressurised air to the patient.

12. A positioning and stabilising structure according to aspect 1 or aspect 2, comprising a pair of lower textile portions adapted to couple to each other, and/or to an intermediate structure, to provide said force.

13. A positioning and stabilising structure according to aspect 12, wherein the intermediate structure is a harness that, in use, retains the seal-forming structure in the therapeutically effective position.

14. A positioning and stabilising structure according to aspect 12, wherein the intermediate structure comprises the seal-forming structure, or part thereof.

15. A positioning and stabilising structure according to any one of aspects 1 to 14, wherein at least one said lower textile portion comprises one or more rigidized sections.

16. A positioning and stabilising structure according to aspect 15, wherein the at least one said lower textile portion has a higher rigidity in an intermediate section thereof than at its ends.

17. A positioning and stabilising structure according to any one of aspects 1 to 16, comprising at least one sensor provided in or on the upper textile portion and/or one or more lower textile portions.

18. A positioning and stabilising structure according to aspect 17, comprising at least one actuator provided in or on the upper textile portion and/or one or more lower textile portions.

19. A positioning and stabilising structure according to aspect 18, wherein the at least one sensor and/or the at least one actuator is partly exposed to ambient at an exterior surface of the upper textile portion or the one or more lower textile portions; and/or is partly exposed at a patient-contacting surface of the upper textile portion or the one or more lower textile portions, so as to contact skin of the patient in use.

20. A positioning and stabilising structure according to aspect 18 or aspect 19, wherein the at least one sensor and/or the at least one actuator is at least partly embedded between an outer layer and a patient-contacting layer of the upper textile portion or of one or more lower textile portions.

21. A positioning and stabilising structure according to any one of aspects 18 to 20, wherein the at least one sensor and/or the at least one actuator comprises circuitry that is at least partially formed by one or more conductive threads, and/or one or more conductive ink traces.

22. A positioning and stabilising structure according to any one of aspects 18 to 21, comprising at least one sensor-retaining structure for attachment of respective ones of the at least one sensors and/or the at least one actuators.

23. A positioning and stabilising structure according to aspect 22, wherein the at least one sensor-retaining structure comprises at least one pocket to accommodate the at least one sensor and/or the at least one actuator.

24. A positioning and stabilising structure according to any one of aspects 18 to 23, comprising a wireless communications interface for transmitting data from the at least one sensor to one or more external computing devices, and/or for receiving data at the at least one actuator from the one or more external computing devices.

25. A positioning and stabilising structure according to any one of aspects 18 to 24, wherein the at least one sensor and/or the at least one actuator comprises one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

26. A patient interface comprising:

a plenum chamber pressurisable to a therapeutic pressure of at least 6 cmH$_2$O above ambient air pressure, said plenum chamber including a plenum chamber inlet port sized and structured to receive a flow of air at the therapeutic pressure for breathing by a patient,

a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and

a positioning and stabilising structure to provide a force to hold the seal-forming structure in a therapeutically effective position on the patient's head;

wherein the positioning and stabilising structure comprises:

a front section and a rear section forming a continuous loop of material, the front section forming a first bifurcated section with a first part and a second part;

an upper textile portion comprising a resilient circumferential band for fitting to a patient's head in use, the upper textile portion formed from the rear section and from the first part; and

at least one lower textile portion formed from the second part and being movably connected to the upper textile portion;

wherein at least a first lower textile portion of the at least one lower textile portion is stretchable relative to the upper textile portion, and is constructed and arranged to provide a force to hold a seal-forming structure of the patient interface in a therapeutically effective position on the patient's head;

wherein the first lower textile portion is movable between a first position and a second position, the first lower textile portion configured to be proximate to the first part and to overlay the patient's frontal bone in the first position, and the first lower textile portion configured to be distal to the first part and overlay the patient's cheeks in the second position.

27. A patient interface according to aspect 26, wherein the first lower textile portion is integral with the upper textile portion.

28. A patient interface according to aspect 26 or aspect 27, wherein the positioning and stabilising structure is in the form of a headband.

29. A patient interface according to any one of aspects 26 to 28, wherein the rear section of the headband comprises a second bifurcated section comprising a second part of the upper textile portion and further comprising a second lower textile portion.

30. A positioning and stabilising structure according to aspect 29, wherein the second part of the upper textile portion and the second lower textile portion are configured to overlay the patient's occipital bone in the first position, and wherein the second part of the upper textile portion is movable away from the second lower textile portion to overlay the parietal bone in the second position.

31. A positioning and stabilising structure according to aspect 29 or aspect 30, wherein the front section and the rear section combine to form an X-shape in the second position.

32. A patient interface according to any one of aspects 26 to 31, wherein the first lower textile portion is a seal retention band that is resiliently stretchable along at least part of its length, and is adapted to engage with an outer surface of the plenum chamber or the seal-forming structure to hold the seal-forming structure in the therapeutically effective position.

33. A patient interface according to aspect 32, wherein the seal retention band is more stretchable than the upper textile portion.

34. A patient interface according to aspect 32 or aspect 33, wherein the seal retention band is received in a channel of the patient interface.

35. A patient interface according to aspect 34, wherein the channel is formed in an external surface of the plenum chamber.

36. A patient interface according to any one of aspects 32 to 35, wherein the seal retention band comprises a port for coupling the plenum chamber inlet port to an air circuit for supplying pressurised air to the patient.

37. A patient interface according to aspect 26 or aspect 27, wherein the positioning and stabilising structure comprises a pair of lower textile portions adapted to couple to each other, and/or to an intermediate structure, to provide said force.

38. A patient interface according to aspect 37, wherein the intermediate structure is a harness that, in use, engages with an external surface of the plenum chamber, or with an external surface of the seal-forming structure.

39. A patient interface according to aspect 37, wherein the intermediate structure comprises the plenum chamber and/or the seal-forming structure, or part thereof.

40. A patient interface according to any one of aspects 26 to 39, wherein at least one said pair of lower textile portions comprises one or more rigidized sections.

41. A patient interface according to aspect 40, wherein the at least one said pair of lower textile portions has a higher rigidity in an intermediate section thereof than at its ends.

42. A patient interface according to any one of aspects 26 to 41, comprising at least one sensor provided in or on the upper textile portion, and/or the at least one of the pair of lower textile portions, and/or the plenum chamber, and/or the seal-forming structure.

43. A patient interface according to aspect 42, comprising at least one actuator provided in or on the upper textile portion, and/or the at least one pair of lower textile portions, and/or the plenum chamber, and/or the seal-forming structure.

44. A patient interface according to aspect 43, wherein at least one sensor and/or at least one actuator is partly exposed to ambient at an exterior surface of the upper textile portion or the at least one pair of lower textile portions; and/or is partly exposed at a patient-contacting surface of the upper textile portion or the at least one pair of lower textile portions, so as to contact skin of the patient in use.

45. A patient interface according to aspect 43 or aspect 44, wherein at least one sensor and/or at least one actuator is at least partly embedded between an outer layer and a patient-contacting layer of the upper textile portion or of the at least one pair of lower textile portions.

46. A patient interface according to any one of aspects 43 to 45, wherein at least one sensor and/or the at least one actuator comprises circuitry that is at least partially formed by one or more conductive threads, and/or one or more conductive ink traces.

47. A patient interface according to any one of aspects 43 to 46, comprising at least one sensor-retaining structure for attachment of respective sensors and/or actuators.

48. A patient interface according to aspect 47, wherein the at least one sensor-retaining structure comprise one or more pockets to accommodate the at least one sensor and/or the at least one actuator.

49. A patient interface according to any one of aspects 43 to 48, comprising a wireless communications interface for transmitting data from the at least one sensor to at least one external computing device, and/or for receiving data at the at least one actuator from the at least one external computing device.

50. A patient interface according to any one of aspects 43 to 49, wherein the at least one sensor and/or the at least one actuator comprise one or more of: an accelerometer; a gyroscope; a humidity sensor; a temperature sensor; a microphone; a camera; a pulse oximeter; an EEG sensor; an EMG sensor; an EOG sensor; a touch sensor; a pressure sensor; a $CO_2$ sensor; a vibration device; and an audio output device.

51. A patient interface according to aspect 50, wherein the at least one sensor comprises at least one pressure sensor in fluid communication with a plenum chamber.

52. A patient interface according to any one of aspects 26 to 51, wherein the plenum chamber comprises a shell and has a shell inside surface and shell outside surface, and wherein the shell inside surface is arranged to be at said therapeutic pressure in use, and said shell outside surface is arranged to be at ambient pressure in use.

53. A patient interface according to aspect 52, wherein at least one of the pair of lower textile portions engages with at least part of the shell outside surface to hold the seal-forming structure in the therapeutically effective position.

54. A patient interface according to aspect 53 when appended to aspect 34, wherein the channel is formed in the shell outside surface.

55. A patient interface according to any one of aspects 52 to 54, wherein the shell is constructed from a hard plastic material.

56. A patient interface according to any one of aspects 52 to 54, wherein the shell is semi-rigid and/or resilient.

57. A patient interface according to any one of aspects 52 to 56, wherein the shell is constructed from a transparent material.

58. A patient interface according to any one of aspects 52 to 57, wherein the shell is coupled to the seal-forming structure.

59. A positioning and stabilising structure for a patient interface, comprising:

a headband formed at least partly from a textile material and having an upper textile portion movably connected to a first lower textile portion, the headband comprising at least one sensor provided in or on the upper textile portion and/or the first lower textile portion;

wherein the headband is wearable on a patient's head in a first configuration in which the first lower textile portion is proximate to the upper textile portion and configured to overlay the patient's forehead, and a second configuration in which the first lower textile portion is separated from the upper textile portion and provides a force to hold a seal-forming structure of the patient interface in a therapeutically effective position on the patient's head, the first lower textile portion configured to overlay the patient's cheeks in the second configuration.

60. A positioning and stabilising structure according to aspect 59, comprising at least one actuator provided in or on the upper textile portion and/or the first lower textile portion.

61. A positioning and stabilising structure according to aspect 60, wherein at least one sensor and/or at least one actuator is partly exposed to ambient at an exterior surface of the upper textile portion or the first lower textile portion; and/or is partly exposed at a patient-contacting surface of the upper textile portion or the first lower textile portion, so as to contact skin of the patient in use.

62. A positioning and stabilising structure according to aspect 60 or aspect 61, wherein at least one sensor and/or at least one actuator is at least partly embedded between an outer layer and a patient-contacting layer of the upper textile portion or of the first lower textile portion.

63. A positioning and stabilising structure according to any one of aspects 60 to 62, wherein at least one sensor and/or at least one actuator comprises circuitry that is at least partially formed by one or more conductive threads, and/or one or more conductive ink traces.

64. A positioning and stabilising structure according to any one of aspects 60 to 63, comprising at least one sensor-retaining structure for attachment to the at least one sensor and/or the at least one actuator.

65. A positioning and stabilising structure according to aspect 64, wherein the one or more sensor-retaining structures comprise one or more pockets to accommodate one or more respective sensors or actuators.

66. A positioning and stabilising structure according to any one of aspects 60 to 65, comprising a wireless communications interface for transmitting data from the at least one sensor to the at least one external computing device, and/or for receiving data at the at least one actuator from the at least one external computing device.

67. A positioning and stabilising structure according to any one of aspects 60 to 66, wherein the at least one sensor and/or the at least one actuator comprise one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

68. A positioning and stabilising structure according to any one of aspects 60 to 67, wherein the first lower textile portion is integral with the upper textile portion.

69. A positioning and stabilising structure according to any one of aspects 60 to 68, wherein the headband comprises a first bifurcated section comprising a first part of the upper textile portion and further comprising the first lower textile portion, the first bifurcated section being located at a front of the headband.

70. A positioning and stabilising structure according to aspect 69, wherein the headband comprises a second bifurcated section comprising a second part of the upper textile portion and further comprising a second lower textile portion, the second bifurcated section being located at a rear of the headband.

71. A positioning and stabilising structure according to any one of aspects 59 to 70, wherein the first lower textile portion is a seal retention band that is resiliently stretchable along at least part of its length, and is adapted to engage with an outer surface of the patient interface to hold the seal-forming structure in the therapeutically effective position.

72. A positioning and stabilising structure according to aspect 71, wherein the seal retention band is more stretchable than the upper textile portion.

73. A positioning and stabilising structure according to aspect 71 or aspect 72, wherein the seal retention band is adapted to be received in a channel of the patient interface.

74. A positioning and stabilising structure according to aspect 73, wherein the channel is formed in a plenum chamber of the patient interface.

75. A positioning and stabilising structure according to any one of aspects 71 to 74, wherein the seal retention band comprises a port for coupling the seal-forming structure to an air circuit for supplying pressurised air to the patient.

76. A positioning and stabilising structure according to any one of aspects 59 to 75, wherein the first lower textile portion comprises one or more rigidized sections.

77. A positioning and stabilising structure according to aspect 76, wherein the first lower textile portion has a higher rigidity in an intermediate section thereof than at its ends.

78. A positioning and stabilising structure according to any one of aspects 59 to 77, wherein at least one resilient hook is located on the first lower textile portion, the at least one resilient hook is configured to connect to one or more projections or recesses on a plenum chamber and/or a seal-forming structure.

79. A patient interface comprising:

a plenum chamber pressurisable to a therapeutic pressure of at least 6 cmH$_2$O above ambient air pressure, said plenum chamber including a plenum chamber inlet port sized and structured to receive a flow of air at the therapeutic pressure for breathing by a patient,
a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and
a positioning and stabilising structure according to any one of aspects 58 to 78.

80. A patient interface according to aspect 79, further comprising one or more sensors positioned in or on an internal surface of the plenum chamber.

81. A patient interface according to aspect 80, wherein the one or more sensors comprise one or more of: a pressure sensor; a humidity sensor; a temperature sensor; and a CO$_2$ sensor.

82. A system for diagnosis and/or monitoring of a respiratory disorder, comprising: a patient interface according to any one of aspects, 42 to 58 or 79 to 81; and at least one computing device in communication with the patient interface to receive data from the one or more sensors of the patient interface.

83. A system for treating a respiratory disorder in a patient, the system comprising:

a patient interface according to any one of aspects 42 to 58 or 79 to 81;
a pressure generator configured to generate a flow of air to the patient interface for treating the respiratory disorder; and
a controller configured to control the pressure generator to adjust the flow of air based on at least one signal received from the at least one sensor of the patient interface.

84. A system according to aspect 83, wherein the at least one sensor of the patient interface comprises EMG and/or EOG sensors, and wherein the controller is configured to: analyse the at least one signal to detect a sleep stage of the patient; and to control the pressure generator to adjust the flow of air based on the sleep stage, and/or to activate one or more audio devices to produce sleep-enhancing noise.

85. A system according to aspect 83 or aspect 84, wherein: the at least one sensor of the patient interface comprises a microphone; and the controller is configured to: analyse a signal from the microphone to detect snoring; and to control the pressure generator to adjust the flow of air based on the detected signals.

86. A system according to any one of aspects, 83 to 85, wherein the at least one sensor comprises a humidity sensor and a temperature sensor inside the plenum chamber; and wherein the controller is configured to control the pressure generator to adjust the flow of air in accordance with signals from the humidity sensor and temperature sensor, to reduce or prevent condensate accumulation.

87. A system according to aspect 86, further comprising a humidifier, wherein the controller is configured to control the humidifier in accordance with signals from the humidity sensor and temperature sensor, to reduce or prevent condensate accumulation.

88. A system according to any one of aspects 83 to 88, wherein the at least one sensor comprises a $CO_2$ sensor inside the plenum chamber; and wherein the controller is configured to: detect whether a $CO_2$ level measured by the $CO_2$ sensor is above a threshold; and to control the patient interface and/or the pressure generator to reduce the $CO_2$ level based on the detected signals.

89. A system according to aspect 88, wherein the controller is configured to send a signal to the patient interface to open an electromechanical vent that is in communication with the plenum chamber, to thereby allow higher flushing of air from the plenum chamber.

90. A system according to aspect 88 or aspect 89, wherein the controller is configured to control the pressure generator to increase the flow to flush out $CO_2$.

91. A system according to any one of aspects 83 to 90 when appended to aspect 44, wherein: the one or more actuators of the patient interface comprise a haptic feedback element.

92. A system according to aspect 91, wherein the haptic feedback element is located in a region of the positioning and stabilising structure that overlies a temple region of the patient's head when in use.

93. A system according to aspect 91 or aspect 92, wherein the at least one sensor comprises a pulse oximeter; and wherein the controller is configured to: receive a heart rate measurement from the pulse oximeter; and responsive to a detection that the heart rate measurement exceeds a threshold, control the haptic feedback element to deliver vibrations to the patient at a rate that is lower than the heart rate measurement.

94. A system according to any one of aspects 91 to 93, wherein the controller is configured to: analyse the at least one signal to detect sleeping position and/or whether a number of apnea and/or hypopnea events of the patient exceeds a threshold; and based on the detection, send a control signal to the haptic feedback element to deliver a tactile stimulus to the patient.

95. A system according to any one of aspects 83 to 94, wherein the at least one sensor comprises an accelerometer; and wherein the controller is configured to: analyse a signal from the accelerometer to detect a position of the patient; and to control the pressure generator to adjust the flow of air based on the detection of the position.

96. A system according to aspect 95, wherein:

when the detected signal indicates that the patient is lying on their back, the pressure is increased slowly;

when the detected signal indicates that the patient is in a side sleeping position, the pressure is lowered; and

when the detected signal indicates that the patient is in an upright position, the flow and pressure are lowered to below the therapeutic pressure.

97. A system according to any one of aspects 83 to 96, wherein the at least one sensor comprises a gyroscope; and wherein the controller is configured to: analyse a signal from the gyroscope to detect movements of the patient; and to control the pressure generator to adjust the flow of air based on the detection of the movements.

98. A system according to aspect 97 wherein:

when the detected signal of the movements indicates a high degree of movement, the controller is configured to control the pressure generator to adjust the pressure to below the therapeutic pressure; and

when the detected signal of the movements indicates a low degree of movement, the controller is configured to control the pressure generator to gradually adjust the pressure towards the therapeutic pressure.

**Claims**

1. A positioning and stabilising structure (11300) for a patient interface, comprising:

   a headband formed at least partly from a textile material and having an upper textile portion (11310) movably connected to a first lower textile portion (11320), the headband comprising at least one sensor (11354, 11358, 11365) provided in or on the upper textile portion (11310) and/or the first lower textile portion (11320);
   wherein the headband is wearable on a patient's head in a first configuration in which the first lower textile portion (11320) is proximate to the upper textile portion (11310) and configured to overlay the patient's forehead, and a second configuration in which the first lower textile portion (11320) is separated from the upper textile portion (11310) and provides a force to hold a seal-forming structure of the patient interface in a therapeutically effective position on the patient's head, the first lower textile portion (11320) configured to overlay the patient's cheeks in the second configuration.

2. A positioning and stabilising structure (11300) according to claim 1, comprising at least one actuator (11360) provided in or on the upper textile portion (11310) and/or the first lower textile portion (11320).

3. A positioning and stabilising structure (11300) according to claim 2, wherein the at least one sensor (11354, 11358, 11365) and/or the at least one actuator (11360) is partly exposed to ambient at an exterior surface of the upper textile portion (11310) or the first lower textile portion (11320), and/or is partly exposed at a patient-contacting surface of the upper textile portion (11310) or the first lower textile portion (11320), so as to contact skin of the patient in use.

4. A positioning and stabilising structure (11300) according to claim 2 or claim 3, wherein the at least one sensor (11354, 11358, 11365) and/or the at least one actuator (11360) is at least partly embedded between an outer layer and a patient-contacting layer of the upper textile portion (11310) or of the first lower textile portion, and/or
   wherein the at least one sensor (11354, 11358, 11365) and/or the at least one actuator (11360) comprises circuitry that is at least partially formed by one or more conductive threads, and/or one or more conductive ink traces.

5. A positioning and stabilising structure (11300) according to any one of claims 2 to 4, comprising at least one sensor-retaining structure for attachment to the at least one sensor (11354, 11358, 11365) and/or the at least one actuator (11360).

6. A positioning and stabilising structure (11300) according to claim 5, wherein the one or more sensor-retaining structures comprise one or more pockets to accommodate one or more respective sensors or actuators (11360).

7. A positioning and stabilising structure (11300) according to any one of claims 2 to 6, comprising a wireless communications interface for transmitting data from the at least one sensor (11354, 11358, 11365) to at least one external computing device, and/or for receiving data at the at least one actuator (11360) from the at least one external computing device.

8. A positioning and stabilising structure (11300) according to any one of claims 2 to 7, wherein the at least one sensor (11354, 11358, 11365) and/or the at least one actuator (11360) comprise one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

9. A positioning and stabilising structure (11300) according to any one of claims 2 to 8, wherein the first lower textile portion (11320) is integral with the upper textile portion (11310).

10. A positioning and stabilising structure (11300) according to any one of claims 2 to 9, wherein the headband comprises a first bifurcated section comprising a first part of the upper textile portion (11310) and further comprising the first lower textile portion (11320), the first bifurcated section being located at a front of the headband, and

wherein the headband comprises a second bifurcated section comprising a second part of the upper textile portion (11310) and further comprising a second lower textile portion (11320), the second bifurcated section being located at a rear of the headband.

11. A positioning and stabilising structure (11300) according to any one of claims 1 to 10, wherein the first lower textile portion (11320) is a seal retention band that is resiliently stretchable along at least part of its length, and is adapted to engage with an outer surface of the patient interface to hold the seal-forming structure in the therapeutically effective position.

12. A positioning and stabilising structure (11300) according to claim 11, wherein the seal retention band is more stretchable than the upper textile portion, and/or

wherein the seal retention band is adapted to be received in a channel of the patient interface, the channel being formed in a plenum chamber of the patient interface, and/or

wherein the seal retention band comprises a port for coupling the seal-forming structure to an air circuit for supplying pressurised air to the patient.

13. A positioning and stabilising structure (11300) according to any one of claims 1 to 12, wherein the first lower textile portion (11320) comprises one or more rigidized sections.

14. A positioning and stabilising structure (11300) according to claim 13, wherein the first lower textile portion (11320) has a higher rigidity in an intermediate section thereof than at its ends.

15. A positioning and stabilising structure (11300) according to any one of claims 1 to 14, wherein at least one resilient hook is located on the first lower textile portion (11320), the at least one resilient hook is configured to connect to one or more projections or recesses on a plenum chamber and/or a seal-forming structure.

**FIG. 1A**

FIG. 1B

FIG. 1C

EP 4 609 903 A2

Nasal cavity

Oral cavity

Larynx

Vocal folds

Oesophagus

Trachea

Bronchus

Lung

Heart

Diaphragm

Alveolar sacs

**FIG. 2A**

**FIG. 2B**

FIG. 2C

FIG. 2D

Coronal plane

Superior

Anterior — Posterior

Inferior

Frankfort horizontal

Nasolabial angle

**FIG. 2E**

73

FIG. 2F

FIG. 2G

Epidermis

Adipose tissue

Nasal bone

Lateral cartilage

Septum cartilage

Greater alar cartilage

Frontal process of maxilla

Lesser alar cartilage

Fibrofatty tissue

FIG. 2H

Frontal sinus

Nasal bone

Septum cartilage

Medial crus of greater alar cartilage

Anterior nasal spine

FIG. 2I

Frontal bone
Sphenoid bone
Nasal bone
Zygomatic bone
Maxilla
Masseter m.
Mandible
Mental protuberance
Digastricus m.
Sternocleidomastoid m.
Concha

Parietal bone
Temporal bone
Occipital bone
Trapezius m.

**FIG. 2J**

**FIG. 2K**

EP 4 609 903 A2

Frontal bone

Supraorbital foramen

Nasal bones

Septal cartilage

Lateral cartilage

Sesamoid cartilage

Greater alar cartilage

Medial crus of greater alar cartilage

Anterior nasal spine

Infraorbital foramen

Lesser nasal cartilage

Alar fibrofatty tissue

Septal cartilage

**FIG. 2L**

FIG. 3A

FIG. 3B — Relatively Large Positive Curvature

FIG. 3C — Relatively Small Positive Curvature

FIG. 3D — Zero Curvature

FIG. 3E — Relatively Small Negative Curvature

FIG. 3F — Relatively Large Negative Curvature

**Legend (FIG. 3G):**
+ = Positive Curvature
− = Negative Curvature
↑ = Outward Normal

Saddle Region +
Dome Region
Saddle Region −
Exterior Surface
Edge of Surface
Dome Region

**FIG. 3G**

**Legend (FIG. 3H):**
+ = Positive Curvature
− = Negative Curvature
↑ = Outward Normal

Saddle Region
Dome Region
A Path on Surface
Edge of Surface
Straight Line Distance
Exterior Surface
Saddle Region

A
B

**FIG. 3H**

FIG. 3J      Curve      FIG. 3J

**FIG. 3I**

Surface

Surface

**FIG. 3J**

**FIG. 3K**

FIG. 3M

FIG. 3M

**FIG. 3L**

FIG. 3N      FIG. 3N

Interior surface

Interior
surface

**FIG. 3M**

**FIG. 3N**

**Left-hand rule**

Binormal(B)

Osculating plane

Tangent(T)

Normal(N)

## FIG. 3O

**Right-hand rule**

Binormal(B)

Osculating plane

Tangent(T)

Normal(N)

## FIG. 3P

**Left ear helix**

## FIG. 3Q

T2

B

B

N

N

T

T

T

T1

**Right-hand helix**
**Right-hand positive**

## FIG. 3S

**Right ear helix**

## FIG. 3R

Right-hand negative
(=left-hand positive)

Right-hand positive

Right-hand negative

Right-hand positive

## FIG. 3T

**FIG. 3U**

**FIG. 3V**

**FIG. 3W**

**FIG. 3X**

6302

6310

6320

6314

6324

6312

6332

6322    6304

6300

**FIG. 4A**

6000

6310

6314

6312

6304

6200

6320

6210

6300

6600

6100

4170

**FIG. 4B**

**FIG. 4C**

**FIG. 4D**

6310

6320

6320

6304

**FIG. 4E**

6310

6320

6320

6304

**FIG. 4F**

**FIG. 5A**

**FIG. 5B**

**FIG. 6**

**FIG. 7A**

**FIG. 7B**

9310

9300

9326

9100

9200

9000

9304

9328

9330

9349

9342

9600

9329

4170

# FIG. 7C

**FIG. 8A**

**FIG. 8B**

**FIG. 9A**

**FIG. 9B**

**FIG. 9C**

**FIG. 10A**

**FIG. 10B**

**FIG. 10C**

4300

4310

Preprocessing

Interface pressure estimation

Vent flow rate estimation

Leak flow rate estimation

4312

4314

Respiratory flow rate estimation

4316

4318

4321

4320

Therapy engine

4322

4323

Phase determination

Waveform determination

Ventilation determination

Flow limitation determination

Apnea / hypopnea determination

Snore determination

4324

4325

4326

Airway patency determination

Target ventilation determination

Therapy parameter determination

4327

4328

4329

4340

Fault condition detection

Therapy control

4330

FIG. 10D

FIG. 10E

**FIG. 11A**

**FIG. 11B**

**FIG. 11C**

**EP 4 609 903 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SG 10202006315Y
- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- WO 1998034665 A
- WO 2000078381 A
- US 6581594 B
- US 20090050156 A
- US 20090044808
- US 7866944 B
- US 8638014 B
- US 8636479 B
- WO 2013020167 A
- US 8733349 B
- WO 2012171072 A
- US 20090065007 A

**Non-patent literature cited in the description**

- **JOHN B. WEST**. Respiratory Physiology. Lippincott Williams & Wilkins, 2012